# EUROPEAN PATENT SPECIFICATION

(11) **EP 0 680 514 B1**
(45) Date of publication and mention of the grant of the patent: **22.10.2003**
(21) Application number: 94906052.9
(22) Date of filing: 06.01.1994
(51) Int. Cl.: C12N 15/82, A01H 5/00, A01N 65/00

(54) **NEMATODE-RESISTANT TRANSGENIC PLANTS**
NEMATOD RESISTENTE TRANSGENE PFLANZEN
PLANTES TRANSGENIQUE RESISTANTES AUX NEMATODES

(30) Priority: 21.01.1993 US 7998
(43) Date of publication of application: 08.11.1995
(73) Proprietor: NORTH CAROLINA STATE UNIVERSITY, Raleigh, North Carolina 27695-7003 (US)
(72) Inventor: CONKLING, Mark A., Fuquay-Varina, NC 27526 (US); OPPERMAN, Charles H., Raleigh, NC 27615 (US); ACEDO, Gregoria N., Durham, NC 27713 (US); SONG, Wen, Raleigh, NC 27607 (US)
(74) Representative: Horner, Martin Grenville
(86) International application number: US9400217
(87) International publication number: WO94017194

(56) References cited:
- WO-A-92/04493
- WO-A-92/21757
- WO-A-93/06710
- WO-A-93/10251
- THE PLANT CELL vol. 3 , 1991 pages 371 - 382 Y.T. YAMAMOTO ET AL.; 'Characterization of cis-acting sequences regulating root-specific gene expression in tobacco'
- SCIENCE vol. 263 , 1994 pages 221 - 223 C.H. OPPERMAN ET AL.; 'Root-knot nematode-directed expression of a plant root-specific gene'

### SEQUENCE LISTING

(1) GENERAL INFORMATION:
   (i) APPLICANT: Conkling, Mark A. Opperman, Charles H. Acedo, Gregoria N. Song, Wen
   (ii) TITLE OF INVENTION: Nematode Resistant Transgenic Plants
   (iii) NUMBER OF SEQUENCES: 7
   (iv) CORRESPONDENCE ADDRESS:
      (A) ADDRESSEE: Kenneth D. Sibley; Bell, Seltzer, Park and Gibson
      (B) STREET: Post Office Drawer 34009
      (C) CITY: Charlotte
      (D) STATE: North Carolina
      (E) COUNTRY: U.S.A.
      (F) ZIP: 28234
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Sibley, Kenneth D.
      (B) REGISTRATION NUMBER: 31,665
      (C) REFERENCE/DOCKET NUMBER: 5051-201
   (ix) TELECOMMUNICATION INFORMATION:
      (A) TELEPHONE: 919-881-3140
      (B) TELEFAX: 919-881-3175
      (C) TELEX: 575102
(2) INFORMATION FOR SEQ ID NO:1:
   (i) SEQUENCE CHARACTERISTICS:
      (A) LENGTH: 938 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 47..799
   (ix) FEATURE:
      (A) NAME/KEY: mat_peptide
      (B) LOCATION: 47..796
   (xi) SEQUENCE DESCRIPTION: SEQ ID NO:1:
   CTTAAATTGA GCTTCTTTTG GGGCATTTTT CTAGTGAGAA CTAAAA ATG GTG AGG        55
                                                      Met Val Arg
                                                        1
   ATT GCC TTT GGT AGC ATT GGT GAC TCT TTT AGT GTT GGA TCA TTG AAG      103
   Ile Ala Phe Gly Ser Ile Gly Asp Ser Phe Ser Val Gly Ser Leu Lys
         5                  10                  15
   GCC TAT GTA GCT GAG TTT ATT GCT ACT CTT CTC TTT GTG TTT GCT GGG      151
   Ala Tyr Val Ala Glu Phe Ile Ala Thr Leu Leu Phe Val Phe Ala Gly
    20                  25                  30                  35
   GTT GGG TCT GCT ATA GCT TAT AAT AAA TTG ACA GCA GAT GCA GCT CTT      199
   Val Gly Ser Ala Ile Ala Tyr Asn Lys Leu Thr Ala Asp Ala Ala Leu
                    40                  45                  50
   GAT CCA GCT GGT CTA GTA GCA GTA GCT GTG GCT CAT GCA TTT GCA TTG      247
   Asp Pro Ala Gly Leu Val Ala Val Ala Val Ala His Ala Phe Ala Leu
                55                  60                  65
   TTT GTT GGG GTT TCC ATA GCA GCC AAT ATT TCA GGT GGC CAT TTG AAT      295
   Phe Val Gly Val Ser Ile Ala Ala Asn Ile Ser Gly Gly His Leu Asn
            70                  75                  80
   CCA GCT GTC ACT TTG GGA TTG GCT GTT GGT GGA AAC ATC ACC ATC TTG      343
   Pro Ala Val Thr Leu Gly Leu Ala Val Gly Gly Asn Ile Thr Ile Leu
        85                  90                  95
   ACT GGC TTC TTC TAC TGG ATT GCC CAA TTG CTT GGC TCC ACA GTT GCT      391
   Thr Gly Phe Phe Tyr Trp Ile Ala Gln Leu Leu Gly Ser Thr Val Ala
   100                 105                 110                 115
   TGC CTC CTC CTC AAA TAC GTT ACT AAT GGA TTG GCT GTT CCA ACC CAT      439
   Cys Leu Leu Leu Lys Tyr Val Thr Asn Gly Leu Ala Val Pro Thr His
                   120                 125                 130
   GGA GTT GCT GCT GGG CTC AAT GGA TTA CAA GGA GTG GTG ATG GAG ATA      487
   Gly Val Ala Ala Gly Leu Asn Gly Leu Gln Gly Val Val Met Glu Ile
               135                 140                 145
   ATC ATA ACC TTT GCA CTG GTC TAC ACT GTT TAT GCA ACA GCA GCA GAC      535
   Ile Ile Thr Phe Ala Leu Val Tyr Thr Val Tyr Ala Thr Ala Ala Asp
           150                 155                 160
   CCT AAA AAG GGC TCA CTT GGA ACC ATT GCA CCC ATT GCA ATT GGG TTC     583
   Pro Lys Lys Gly Ser Leu Gly Thr Ile Ala Pro Ile Ala Ile Gly Phe
       165                 170                 175
   ATT GTT GGG GCC AAC ATT TTG GCA GCT GGT CCA TTC AGT GGT GGG TCA     631
   Ile Val Gly Ala Asn Ile Leu Ala Ala Gly Pro Phe Ser Gly Gly Ser
   180                 185                 190                 195
   ATG AAC CCA GCT CGA TCA TTT GGG CCA GCT GTG GTT GCA GGA GAC TTT     679
   Met Asn Pro Ala Arg Ser Phe Gly Pro Ala Val Val Ala Gly Asp Phe
                   200                 205                 210
   TCT CAA AAC TGG ATC TAT TGG GCC GGC CCA CTC ATT GGT GGA GGA TTA     727
   Ser Gln Asn Trp Ile Tyr Trp Ala Gly Pro Leu Ile Gly Gly Gly Leu
               215                 220                 225
   GCT GGG TTT ATT TAT GGA GAT GTC TTT ATT GGA TGC CAC ACC CCA CTT     775
   Ala Gly Phe Ile Tyr Gly Asp Val Phe Ile Gly Cys His Thr Pro Leu
           230                 235                 240
   CCA ACC TCA GAA GAC TAT GCT TAAAACTTAA AAGAAGACAA GTCTGTCTTC     826
   Pro Thr Ser Glu Asp Tyr Ala
       245                 250
   AATGTTTCTT TGTGTGTTTT CAAATGCAAT GTTGATTTTT AATTTAAGCT TTGTATATTA     886
   TGCTATGCAA CAAGTTTGTT TCCAATGAAA TATCATGTTT TGGTTTCTTT TG     938
(2) INFORMATION FOR SEQ ID NO:2:
   (i) SEQUENCE CHARACTERISTICS:
      (A) LENGTH: 250 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Val Arg Ile Ala Phe Gly Ser Ile Gly Asp Ser Phe Ser Val Gly
   1               5                   10                  15
   Ser Leu Lys Ala Tyr Val Ala Glu Phe Ile Ala Thr Leu Leu Phe Val
               20                  25                  30
   Phe Ala Gly Val Gly Ser Ala Ile Ala Tyr Asn Lys Leu Thr Ala Asp
           35                  40                  45
   Ala Ala Leu Asp Pro Ala Gly Leu Val Ala Val Ala Val Ala His Ala
       50                  55                  60
   Phe Ala Leu Phe Val Gly Val Ser Ile Ala Ala Asn Ile Ser Gly Gly
   65                  70                  75                  80
   His Leu Asn Pro Ala Val Thr Leu Gly Leu Ala Val Gly Gly Asn Ile
   85 90 95
   Thr Ile Leu Thr Gly Phe Phe Tyr Trp Ile Ala Gln Leu Leu Gly Ser
   100 105 110
   Thr Val Ala Cys Leu Leu Leu Lys Tyr Val Thr Asn Gly Leu Ala Val
   115 120 125
   Pro Thr His Gly Val Ala Ala Gly Leu Asn Gly Leu Gln Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ala
   195 200 205
   Gly Asp Phe Ser Gln Asn Trp Ile Tyr Trp Ala Gly Pro Leu Ile Gly
   210 215 220
   Gly Gly Leu Ala Gly Phe Ile Tyr Gly Asp Val Phe Ile Gly Cys His
   225 230 235 240
   Thr Pro Leu Pro Thr Ser Glu Asp Tyr Ala
   245 250
(2) INFORMATION FOR SEQ ID NO:3:
   (i) SEQUENCE CHARACTERISTICS:
      (A) LENGTH: 938 base pairs
      (B) TYPE: nucleic acid
      (C) STRANDEDNESS: single
      (D) TOPOLOGY: linear
   (ii) MOLECULE TYPE: cDNA
   (iv) ANTI-SENSE: YES
   (xi) SEQUENCE DESCRIPTION: SEQ ID NO:3:
   CAAAAGAAAC CAAAACATGA TATTTCATTG GAAACAAACT TGTTGCATAG CATAATATAC 60
   AAAGCTTAAA TTAAAAATCA ACATTGCATT TGAAAACACA CAAAGAAACA TTGAAGACAG 120
   ACTTGTCTTC TTTTAAGTTT TAAGCATAGT CTTCTGAGGT TGGAAGTGGG GTGTGGCATC 180
   CAATAAAGAC ATCTCCATAA ATAAACCCAG CTAATCCTCC ACCAATGAGT GGGCCGGCCC    240
   AATAGATCCA GTTTTGAGAA AAGTCTCCTG CAACCACAGC TGGCCCAAAT GATCGAGCTG    300
   GGTTCATTGA CCCACCACTG AATGGACCAG CTGCCAAAAT GTTGGCCCCA ACAATGAACC    360
   CAATTGCAAT GGGTGCAATG GTTCCAAGTG AGCCCTTTTT AGGGTCTGCT GCTGTTGCAT    420
   AAACAGTGTA GACCAGTGCA AAGGTTATGA TTATCTCCAT CACCACTCCT TGTAATCCAT    480
   TGAGCCCAGC AGCAACTCCA TGGGTTGGAA CAGCCAATCC ATTAGTAACG TATTTGAGGA    540
   GGAGGCAAGC AACTGTGGAG CCAAGCAATT GGGCAATCCA GTAGAAGAAG CCAGTCAAGA    600
   TGGTGATGTT TCCACCAACA GCCAATCCCA AAGTGACAGC TGGATTCAAA TGGCCACCTG    660
   AAATATTGGC TGCTATGGAA ACCCCAACAA ACAATGCAAA TGCATGAGCC ACAGCTACTG    720
   CTACTAGACC AGCTGGATCA AGAGCTGCAT CTGCTGTCAA TTTATTATAA GCTATAGCAG    780
   ACCCAACCCC AGCAAACACA AAGAGAAGAG TAGCAATAAA CTCAGCTACA TAGGCCTTCA    840
   ATGATCCAAC ACTAAAAGAG TCACCAATGC TACCAAAGGC AATCCTCACC ATTTTTAGTT    900
   CTCACTAGAA AAATGCCCCA AAAGAAGCTC AATTTAAG                            938
(2) INFORMATION FOR SEQ ID NO:4:
   (i) SEQUENCE CHARACTERISTICS:
      (A) LENGTH: 706 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   GTCCTACACA ATGTGAATTT GAATTAGTTT GGTCATACGG TATATCATAT GATTATAAAT     60
   AAAAAAAATT AGCAAAAGAA TATAATTTAT TAAATATTTT ACACCATACC AAACACAACC    120
   GCATTATATA TAATCTTAAT TATCATTATC ACCAGCATCA ACATTATAAT GATTCCCCTA    180
   TGCGTTGGAA CGTCATTATA GTTATTCTAA ACAAGAAAGA AATTTGTTCT TGACATCAGA    240
   CATCTAGTAT TATAACTCTA GTGGAGCTTA CCTTTTCTTT TCCTTCTTTT TTTTCTTCTT    300
   AAAAAAATTA TCACTTTTTA AATCTTGTAT ATTAGTTAAG CTTATCTAAA CAAAGTTTTA    360
   AATTCATTTC TTAAACGTCC ATTACAATGT AATATAACTT AGTCGTCTCA ATTAAACCAT    420
   TAATGTGAAA TATAAATCAA AAAAAGCCAA AGGGCGGTGG GACGGCGCCA ATCATTTGTC    480
   CTAGTCCACT CAAATAAGGC CCATGGTCGG CAAAACCAAA CACAAAATGT GTTATTTTTA 540
   ATTTTTTCCT CTTTTATTGT TAAAGTTGCA AAATGTGTTA TTTTTGGTAA GACCCTATGG 600
   ATATATAAAG ACAGGTTATG TGAAACTTGG AAAACCATCA AGTTTTAAGC AAAACCCTCT 660
   TAAGAACTTA AATTGAGCTT CTTTTGGGGC ATTTTTCTAG TGAGAA 706
(2) INFORMATION FOR SEQ ID NO:5:
   (i) SEQUENCE CHARACTERISTICS:
      (A) LENGTH: 368 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   AGCTTATCTA AACAAAGTTT TAAATTCATT TCTTAAACGT CCATTACAAT GTAATATAAC 60
   TTAGTCGTCT CAATTAAACC ATTAATGTGA AATATAAATC AAAAAAAGCC AAAGGGCGGT 120
   GGGACGGCGC CAATCATTTG TCCTAGTCCA CTCAAATAAG GCCCATGGTC GGCAAAACCA 180
   AACACAAAAT GTGTTATTTT TAATTTTTTC CTCTTTTATT GTTAAAGTTG CAAAATGTGT 240
   TATTTTTGGT AAGACCCTAT GGATATATAA AGACAGGTTA TGTGAAACTT GGAAAACCAT 300
   CAAGTTTTAA GCAAAACCCT CTTAAGAACT TAAATTGAGC TTCTTTTGGG GCATTTTTCT 360
   AGTGAGAA 368
(2) INFORMATION FOR SEQ ID NO:6:
   (i) SEQUENCE CHARACTERISTICS:
      (A) LENGTH: 3426 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: promoter
      (B) LOCATION: 1..1877
   (ix) FEATURE:
      (A) NAME/KEY: exon
      (B) LOCATION: 1954..2079
   (ix) FEATURE:
      (A) NAME/KEY: intron
      (B) LOCATION: 2080..2375
   (ix) FEATURE:
      (A) NAME/KEY: exon
      (B) LOCATION: 2376..2627
   (ix) FEATURE:
      (A) NAME/KEY: intron
      (B) LOCATION: 2628..2912
   (ix) FEATURE:
      (A) NAME/KEY: exon
      (B) LOCATION: 2913..3284
   (ix) FEATURE:
      (A) NAME/KEY: 5'UTR
      (B) LOCATION: 1878..1953
   (ix) FEATURE:
      (A) NAME/KEY: CDS
      (B) LOCATION: join(1954..2079, 2376..2627, 2913..3284)
   (xi) SEQUENCE DESCRIPTION: SEQ ID NO:6:
   GGATCCCCCT CTTTTATAAT AGAGGGTCAT TACTTTATTT ACAATAAAAT AATAAAATAA   60
   AGCATATAGT GGAGGACCCA TGATGACTTG TTTCTTCCTC GATTTTCGCC GAGATTCTCT  120
   CCCATAGTGC GGTTGCAACG GCCCTTGTCT GCGAGCTCGA TACTGGTTCG AGCTCGGCAT  180
   TGGACCGAGC CCTCGACCTT GGTCCGAGCT CGATTCTGAC TTGGGGTCTC GGTATTCGGG  240
   GTGAGTGTTG GTCGGTCTAT GCATCTTCGA TAATCTCCGT TTTGCCTCGT AGTTCGATTT  300
   GGATATGAGC TCGATAATGA TACCGAGCTT GTCATTGATC GGTCTTAGAG CTCGAAGTTC  360
   GACGCCTTTA CTTCGGACCT TGACCGAGCT TGTTATGTAG ATATCCTTTG ATCGAAACAT  420
   TATCGTTTTG ACCAATCCGT ACGACTGACT CAAATCGATT TGACCGCACA CAAGATTATT  480
   TTCGAAAGAC CCTCGACGTC TTGGAGTATA AAATAATTTA GTAAAGAGAG TAATTGTTCG  540
   TTAAAAATCT TGACACCATT CCAAGCATAC CCCTTATTGT ACTTCAATTA ATTATCATTA  600
   TATCAGCATA AACATTATAA TAAGTTTCTT GCGTGTTGGA ACGTCATTTT AGTTATTCTA  660
   AAGAGGAAAT AGTTTCTTTT TTGCTCATGA CATCAGACAT CTGGACTACT ATACTGGAGT  720
   TTACCTTTTC TTCTCCTCTT TTTCTTATTG TTCCTCTAAA AAAAATTATC ACTTTTTAAA  780
   TGCATTAGTT AAACTTATCT CAACAACGTT TAAAATTCAT TTCTTGAATG CCCATTACAA  840
   TGTAATAGTA TAACTTAATT AGTCGTCTCC ATGAACCATT AATACGTACG GAGTAATATA  900
   AAACACCATT GGGGAGTTCA ATTTGCAATA ATTTCTTGCA AAAATGTAAA GTACCTTTTT    960
   GTTCTTGCAA AATTTTACAA ATAAAAATTT GCAGCTCTTT TTTTTCTCTC TCTCCAAATA   1020
   CTAGCTCAAA ACCCACAAAT ATTTTTGAAT TTATGGCATA CTTTTAGAAT GCGTTTGATG   1080
   CAACTATTTT CCTTTAGGAA ATATTCACAA CAATCTAAGA CAATCAAAAA GTAGAAAATA   1140
   GTTTGTAAAA AGGGATGTGG AGGACATCTT AATCAAATAT TTTCAGTTTA AAACTTGAAA   1200
   ATGAAAAAAC ACCCGAAAGG AAATGATTCG TTCTTTAATA TGTCCTACAC AATGTGAATT   1260
   TGAATTAGTT TGGTCATACG GTATATCATA TGATTATAAA TAAAAAAAAT TAGCAAAAGA   1320
   ATATAATTTA TTAAATATTT TACACCATAC CAAACACAAC CGCATTATAT ATAATCTTAA   1380
   TTATCATTAT CACCAGCATC AACATTATAA TGATTCCCCT ATGCGTTGGA ACGTCATTAT   1440
   AGTTATTCTA AACAAGAAAG AAATTTGTTC TTGACATCAG ACATCTAGTA TTATAACTCT   1500
   AGTGGAGCTT ACCTTTTCTT TTCCTTCTTT TTTTTCTTCT TAAAAAAATT ATCACTTTTT   1560
   AAATCTTGTA TATTAGTTAA GCTTATCTAA ACAAAGTTTT AAATTCATTT CTTAAACGTC   1620
   CATTACAATG TAATATAACT TAGTCGTCTC AATTAAACCA TTAATGTGAA ATATAAATCA   1680
   AAAAAAGCCA AAGGGCGGTG GGACGGCGCC AATCATTTGT CCTAGTCCAC TCAAATAAGG   1740
   CCCATGGTCG GCAAAACCAA ACACAAAATG TGTTATTTTT AATTTTTTCC TCTTTTATTG   1800
   TTAAAGTTGC AAAATGTGTT ATTTTTGGTA AGACCCTATG GATATATAAA GACAGGTTAT   1860
   GTGAAACTTG GAAAACCATC AAGTTTTAAG CAAAACCCTC TTAAGAACTT AAATTGAGCT   1920
   TCTTTTGGGG CATTTTTCTA GTGAGAACTA AAA ATG GTG AGG ATT GCC TTT GGT    1974
                                        Met Val Arg Ile Ala Phe Gly
                                         1               5
   AGC ATT GGT GAC TCT TTT AGT GTT GGA TCA TTG AAG GCC TAT GTA GCT     2022
   Ser Ile Gly Asp Ser Phe Ser Val Gly Ser Leu Lys Ala Tyr Val Ala
           10                  15                  20
   GAG TTT ATT GCT ACT CTT CTC TTT GTG TTT GCT GGG GTT GGG TCT GCT     2070
   Glu Phe Ile Ala Thr Leu Leu Phe Val Phe Ala Gly Val Gly Ser Ala
       25                  30                  35
   ATA GCT TAT AGTAAGTAAC ACTTCTCTAA TTAAACTTGC ATGCTAACAT             2119
   Ile Ala Tyr
    40
   AAATACTTAA TCTGCTCTAG CACTAAATAG TAAAAAGAGC AATCAGGTGC ACTAAGGTCC   2179
   CATTAATTCG TTATGCACAT GCCACGGAGT CTAGAGAAAG ACTAGACTGG CTCTATCATA   2239
   TTCAATTTTA CCTTACATTT TACTAGATGC CGTTTTCTCA ATCCATAACC GAAAACAACA   2299
   TAACTTTTAC AGTTACACCA AGACTGCCTA ATTAACCTTT TTTTTTTTTT TTTTTGCTTT    2359
   GTGGGGTGAT TTTGTA GAT AAA TTG ACA GCA GAT GCA GCT CTT GAT CCA        2408
                     Asp Lys Leu Thr Ala Asp Ala Ala Leu Asp Pro
                             45                  50
   GCT GGT CTA GTA GCA GTA GCT GTG GCT CAT GCA TTT GCA TTG TTT GTT      2456
   Ala Gly Leu Val Ala Val Ala Val Ala His Ala Phe Ala Leu Phe Val
       55                  60                  65
   GGG GTT TCC ATA GCA GCC AAT ATT TCA GGT GGC CAT TTG AAT CCA GCT      2504
   Gly Val Ser Ile Ala Ala Asn Ile Ser Gly Gly His Leu Asn Pro Ala
   70                  75                  80                  85
   GTA ACT TTG GGA TTG GCT GTT GGT GGA AAC ATC ACC ATC TTG ACT GGC      2552
   Val Thr Leu Gly Leu Ala Val Gly Gly Asn Ile Thr Ile Leu Thr Gly
                   90                  95                  100
   TTC TTC TAC TGG ATT GCC CAA TTG CTT GGC TCC ACA GTT GCT TGC CTC      2600
   Phe Phe Tyr Trp Ile Ala Gln Leu Leu Gly Ser Thr Val Ala Cys Leu
               105                 110                 115
   CTC CTC AAA TAC GTT ACT AAT GGA TTG GTATGTACTG CTATCATTTT            2647
   Leu Leu Lys Tyr Val Thr Asn Gly Leu
           120                 125
   CAATCCATAT TATATGTCTT TTTATATTTT TCACAACTTC AATAAAAAAA CAACTTTACC    2707
   TAAGACCAGC CTAAGCCGTC GTATAGCCGT CCATCCAACC CTTTAAATTA AAAAGAGCCG    2767
   GCATAGTCAT AATATATGTA TATTTCATGT AGAATATTTG TATAATTAGT GTATATTGTA    2827
   CGTATATCGA CTAGAAAAAA ATAAATAATG AATATGACTG TTTATTTGTA ATTGGAGTTG    2887
   GGCCTCATAT GTTGGTTTTT GGCAG GCT GTT CCA ACC CAT GGA GTT GCT GCT      2939
                               Ala Val Pro Thr His Gly Val Ala Ala
                                           130                 135
   GGG CTC AAT GGA TTA CAA GGA GTG GTG ATG GAG ATA ATC ATA ACC TTT      2987
   Gly Leu Asn Gly Leu Gln Gly Val Val Met Glu Ile Ile Ile Thr Phe
                   140                 145                 150
   GCA CTG GTC TAC ACT GTT TAT GCA ACA GCA GCA GAC CCT AAA AAG GGC      3035
   Ala Leu Val Tyr Thr Val Tyr Ala Thr Ala Ala Asp Pro Lys Lys Gly
               155                 160                 165
   TCA CTT GGA ACC ATT GCA CCC ATT GCA ATT GGG TTC ATT GTT GGG GCC      3083
   Ser Leu Gly Thr Ile Ala Pro Ile Ala Ile Gly Phe Ile Val Gly Ala
           170                 175                 180
   AAC ATT TTG GCA GCT GGT CCA TTC AGT GGT GGG TCA ATG AAC CCA GCT      3131
   Asn Ile Leu Ala Ala Gly Pro Phe Ser Gly Gly Ser Met Asn Pro Ala
       185                 190                 195
   CGA TCA TTT GGG CCA GCT GTG GTT GCA GGA GAC TTT TCT CAA AAC TGG      3179
   Arg Ser Phe Gly Pro Ala Val Val Ala Gly Asp Phe Ser Gln Asn Trp
   200                 205                 210                 215
   ATC TAT TGG GCC GGC CCA CTC ATT GGT GGA GGA TTA GCT GGG TTT ATT      3227
   Ile Tyr Trp Ala Gly Pro Leu Ile Gly Gly Gly Leu Ala Gly Phe Ile
                   220                 225                 230
   TAT GGA GAT GTC TTT ATT GGA TGC CAC ACC CCA CTT CCA ACC TCA GAA      3275
   Tyr Gly Asp Val Phe Ile Gly Cys His Thr Pro Leu Pro Thr Ser Glu
               235                 240                 245
   GAC TAT GCT TAAAACTTAA AAGAAGACAA GTCTGTCTTC AATGTTTCTT              3324
   Asp Tyr Ala
           250
   TGTGTGTTTT CAAATGCAAT GTTGATTTTT AATTTAAGCT TTGTATATTA TGCTATGCAA    3384
   CAAGTTTGTT TCCAATGAAA TATCATGTTT TGGTTTCTTT TG                       3426
(2) INFORMATION FOR SEQ ID NO:7:
   (i) SEQUENCE CHARACTERISTICS:
      (A) LENGTH: 250 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Val Arg Ile Ala Phe Gly Ser Ile Gly Asp Ser Phe Ser Val Gly
   1               5                   10                  15
   Ser Leu Lys Ala Tyr Val Ala Glu Phe Ile Ala Thr Leu Leu Phe Val
               20                  25                  30
   Phe Ala Gly Val Gly Ser Ala Ile Ala Tyr Asp Lys Leu Thr Ala Asp
           35                  40                  45
   Ala Ala Leu Asp Pro Ala Gly Leu Val Ala Val Ala Val Ala His Ala
       50                  55                  60
   Phe Ala Leu Phe Val Gly Val Ser Ile Ala Ala Asn Ile Ser Gly Gly
   65                  70                  75                  80
   His Leu Asn Pro Ala Val Thr Leu Gly Leu Ala Val Gly Gly Asn Ile
                   85                  90                  95
   Thr Ile Leu Thr Gly Phe Phe Tyr Trp Ile Ala Gln Leu Leu Gly Ser
               100                 105                 110
   Thr Val Ala Cys Leu Leu Leu Lys Tyr Val Thr Asn Gly Leu Ala Val
           115                 120                 125
   Pro Thr His Gly Val Ala Ala Gly Leu Asn Gly Leu Gln Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ala
   195 200 205
   Gly Asp Phe Ser Gln Asn Trp Ile Tyr Trp Ala Gly Pro Leu Ile Gly
   210 215 220
   Gly Gly Leu Ala Gly Phe Ile Tyr Gly Asp Val Phe Ile Gly Cys His
   225 230 235 240
   Thr Pro Leu Pro Thr Ser Glu Asp Tyr Ala
   245 250

## Description

### Field of the Invention

This invention relates to methods of controlling plant-parasitic nematodes by application of recombinant DNA technology and the production of transgenic plants.

### Background of the Invention

World-wide, plant-parasitic nematodes are among the most devastating pathogens of life sustaining crops. In 1984, nematodes accounted for more than fifty billion dollars (US) in economic losses. The United States' portion of this figure alone is almost six billion dollars. Genetic resistance to certain nematode species is available in some cultivars, but these are restricted in number, and the availability of cultivars with both desirable agronomic features and resistance is limited. In addition, traditional methods for plant breeding require 5-10 years to produce a viable cultivar, while the need for new nematode control tools is immediate and critical.

The major means of nematode control has been the application of chemical nematicides. During 1982, in the United States alone over 100 million pounds of nematicide were applied to crops. Chemical nematicides are generally highly toxic compounds known to cause substantial environmental impact. In the past several years, issues such as ground water contamination, mammalian and avian toxicity, and residues in food have caused much tighter restrictions on the use of chemical nematicides. Unfortunately, in many situations there is no alternative available for growers who rely upon nematicides to protect their crop from root-knot and cyst nematodes. Accordingly, there is a continuing need for new ways to combat nematodes in plants. In WO-A- 9 204 453 nematode resistant plants are described. The genes identified therein are different from the nematode pore protein genes of the present application.

### Summary of the Invention

A first aspect of the present invention is a DNA construct comprising a transcription cassette. The construct comprises, in the 5' to 3' direction, (a) a promoter operable in a plant cell, (b) a DNA comprising at least 15 nucleotides **which are antisense to** a DNA sequence encoding a nematode-inducible transmembrane pore protein or **a DMA, encoding an enzymatic RNA molecule directed against a nematode-inducible transmembrane pore protein** , and (C) optionally, but preferably, a termination signal. The promoter may be one which is constitutively active in plant cells, selectively active in plant root tissue cells, or a nematode-responsive element such as the nematode-responsive element of the Tobacco RB7 (TobRB7) promoter. Such constructs may be carried by a plant transformation vector such as an Agrobacterium tumefaciens vector, which are in turn used to produce recombinant plants.

A second aspect of the present invention is, accordingly, a nematode-resistant transgenic plant. The plant comprises cells containing a DNA construct comprising a transcription cassette as described above.

In particular embodiments of the invention, DNA encoding a nematode-inducible transmembrane pore protein may be selected from the group-consisting of: **(a)** isolated DNA having the sequence given herein as SEQ ID NO:1 (which DNA encodes the nematode-inducible transmembrane pore protein given herein as SEQ ID NO:2) or SEQ ID NO:6 (which is a genomic DNA encoding the nematode-inducible transmembrane pore protein given herein as SEQ ID NO:7, which is the same as SEQ ID NO:2); **(b)** isolated DNA which hybridizes to isolated DNA of (a) above and which encodes a nematode inducible transmembrane pore protein (which isolated DNA is preferably at least 50% homologous with an isolated DNA of (a) above; and which pore protein is preferably at least 60% homologous with a pore protein of (a) above); and **(c)** isolated DNA differing from the isolated DNAs of (a) and (b) above in nucleotide sequence due to the degeneracy of the genetic code, and which encode a nematode-inducible transmembrane pore protein. A specific example of such a DNA, in antisense configuration for carrying out the present invention, is given herein as SEQ ID NO:3.

Additionally, in particular embodiments of the invention, DNA encoding a nematode-responsive element may be selected from the group consisting of: **(i)** isolated DNA having the sequence given herein as SEQ ID NO:5; and (ii) isolated DNA which hybridizes to isolated DNA of (i) above and which encodes a nematode responsive element (which is preferably at least 60% homologous to isolated DNA of (i) above; and which are preferably at least 10 or 15 nucleotides in length) (this definition is intended to include fragments of (i) above which retain activity as nematode-responsive elements).

The foregoing and other objects and aspects of this invention are explained in detail in the drawings herein and the specification set forth below.

### Brief Description of the Drawings

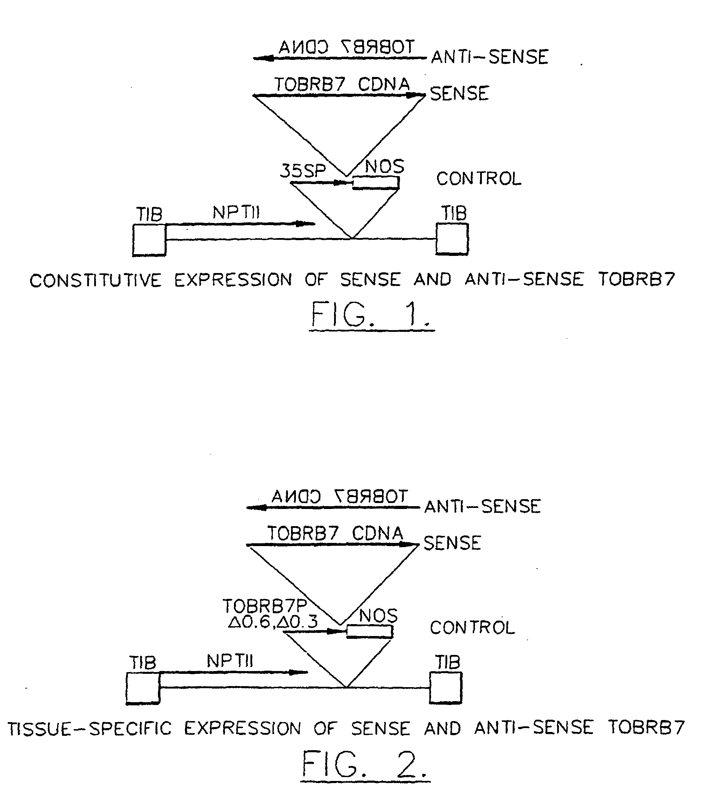

**Figure 1** illustrates a pair of DNA constructs comprising transcription cassettes, one in which the TobRB7 cDNA in sense configuration under the transcriptional control of a CaMV 35S promoter, and the other with a TobRB7 cDNA in antisense configuration under the transcriptional control of a CaMV 35S promoter. A nos 3' termination sequence and a neomycin phosphotransferase II (NPT-II) selectable marker for imparting kanamycin resistance is provided in both cases. The border regions of the Ti plasmid into which the cassette is inserted are indicated as "TiB".
**Figure 2** illustrates transcription cassettes much like those illustrated in Figure 1 above, except that the constitutively active CaMV35S promoter is replaced with either the element TobRB7 Δ0.6 which is selectively active in root tissue cells or the nematode-responsive element TobRB7 Δ0.3.

### Detailed Description of the Invention

The present invention is employed to combat nematodes, particularly the root knot nematodes (*Meloidogyne* spp.) and the cyst nematodes *(Globodera* spp. and *Heterodera* spp.). These nematodes have similar life cycles. Root-knot nematodes are sedentary endoparasites with an extremely intimate and complex relationship to the host plant. The infective second stage juvenile (J2) is free in the soil. Upon location of a host root, the J2 penetrates the root intercellularly in the region just posterior to the root cap and migrates to the developing vascular cylinder. The nematode then orients itself parallel to the cylinder and injects glandular secretions into the plant cells surrounding its head, resulting in the initiation of nematode feeding cells. These 5-7 cells undergo rapid nuclear divisions, increase tremendously in size, and become filled with pores and cell wall invaginations. The feeding site cells, or "giant cells", function as super transfer cells to provide nourishment to the developing nematode. During this time, the nematode loses the ability to move and swells from the normal eel shaped J2 to a large, pear shaped adult female. As the nematode feeds on the giant cells, parthenogenic reproduction results in the the disposition of 300-1000 eggs. This entire process occurs over the span of 20-30 days, and root-knot nematodes may complete as many as 7 generations during a cropping season. The life cycle of the cyst nematode is essentially the same, except that its feeding site is referred to as a "syncytia", and it undergoes sexual reproduction.

Nematode-inducible transmembrane pore proteins are pore proteins the expression of which is increased in cells upon infection of a plant containing the cells by a plant-parasitic nematode at a position adjacent those cells. Increased expression of such pore proteins is required by the nematode in establishing a feeding site capable of passing nutrients from the plant to the nematode. In general, and as explained in greater detail below, DNA encoding nematode-inducible transmembrane pore proteins include DNA which is 50% homologous or more with DNA having the sequence given herein as SEQ ID NO:1 or SEQ ID NO:6. With respect to the protein, DNA encoding nematode-inducible transmembrane pore proteins encode a protein which, in amino acid content, is about 60% homologous or more, or preferably about 70% homologous or more, with the protein having the amino acid sequence given herein as SEQ ID NO:2. Determinations of homology are made with the two sequences (nucleic acid or amino acid) aligned for maximum matching. Gaps in either of the two sequences being matched are allowed in maximizing matching. Gaps lengths of 10 or less are preferred, gap lengths of 5 or less are more preferred, and gap lengths of 2 or less still more preferred.

Differential hybridization procedures are available which allow for the isolation of cDNA clones whose mRNA levels are as low as about 0.05% of poly(A⁺)RNA. *See* M. Conkling et al., *Plant Physiol.* **93**, 1203-1211 (1990). In brief, cDNA libraries are screened using single-stranded cDNA probes of reverse transcribed mRNA from plant tissue (i.e., roots and leaves). For differential screening, a nitrocellulose or nylon membrane is soaked in 5xSSC, placed in a 96 well suction manifold, 150 µL of stationary overnight culture transferred from a master plate to each well, and vacuum applied until all liquid has passed through the filter. 150 µL of denaturing solution (0.5M NaOH, 1.5 M NaCl) is placed in each well using a multiple pipetter and allowed to sit about 3 minutes. Suction is applied as above and the filter removed and neutralized in 0.5 M Tris-HCl (pH 8.0), 1.5 M NaCl. It is then baked 2 hours in *vacua* and incubated with the relevant probes. By using nylon membrane filters and keeping master plates stored at -70°C in 7% DMSO, filters may be screened multiple times with multiple probes and appropriate clones recovered after several years of storage.

For example, to isolate genes whose expression is induced or enhanced by nematode infection, a cDNA library of mRNA isolated from nematode infected tobacco roots is constructed. The roots are staged such that mRNA is isolated at the time of giant cell initiation. The library is then screened by the procedures given above using single stranded cDNA probes of mRMA isolated from nematode-infected and control roots. Those cDNA clones exhibiting differential expression are then used as probes on tobacco genomic Southern blots (to confirm the cDNA corresponds to tobacco and not nematode transcripts) and Northern blots of root RNA from infected and control tissue (to confirm differential expression). Those clones exhibiting differential expression are then used as probes to screen an existing tobacco genomic library. Essentially the same procedure is carried out with plants other than tobacco and nematodes (or other pathogens) other than root-knot nematodes. The procedure is useful for identifying promoters induced by cyst nematodes, in which case the roots are staged such that mRNA is isolated at the time of syncytia initiation. For example, a potato-cyst nematode (*Globodera* spp.) inducible promoter is isolated from potato plants (*Solanum tuberosum*) in accordance with the foregoing procedures.

We have probed a wide variety of dicotyledonous and monocotyledonous plants at low stringency with TobRB7 probes and have found that most (if not all) plants contain a TobRB7 analog. We have already identified by low stringency hybridization such a root-specific cDNA analog from *Arabidopsis thaliana* (AtRB7) (Yamamoto, Cheng, and Conkling 1990 *Nucl. Acids Res.* **18:** *7449).*

Nematode-inducible transmembrane pore proteins employed in carrying out the present invention include proteins homologous to, and having essentially the same biological properties as, the nematode-inducible pore protein Tobacco RB7 disclosed herein as SEQ ID NO:2 (the same as SEQ ID NO:7). This definition is intended to encompass natural allelic variations in the pore protein. Cloned genes employed in carrying out the present invention may code for a nematode-inducible pore protein of any species of origin, including tobacco, soybean, potato, peanuts, pineapple, cotton, and vegetable crops, but preferably encode a nematode-inducible transmembrane pore protein of dicot origin. Thus, DNA sequences which hybridize to DNA of SEQ ID NO:1 or SEQ ID NO:6 and code on expression for a nematode-inducible transmembrane pore protein may also be employed in carrying out the present invention. Conditions which will permit other DNA sequences which code on expression for a pore protein to hybridize to a DNA having the sequence given as SEQ ID NO:1 or SEQ ID NO:6 can be determined in a routine manner. For example, hybridization of such sequences may be carried out under conditions of reduced stringency or even stringent conditions (e.g., conditions represented by a wash stringency of 0.3 M NaCl, 0.03 M sodium citrate, 0.1% SDS at 60°C or even 70°C to DNA having the sequence given as SEQ ID NO:1 or SEQ ID NO:6 herein in a standard in situ hybridization assay. See J. Sambrook et al., Molecular Cloning, A Laboratory Manual (2d Ed. 1989)(Cold Spring Harbor Laboratory)). In general, such sequences will be at least 75% homologous, 80% homologous, 85% homologous, 90% homologous, or even 95% homologous or more with the sequence given herein as SEQ ID NO:1 or SEQ ID NO:6 (in the case of SEQ ID NO:6, which is a genomic sequence, such homology is with respect to the exons alone, though the homology may be considered with respect to both introns and exons). Determinations of homology are made with the two sequences aligned for maximum matching. Gaps in either of the two sequences being matched are allowed in maximizing matching. Gap lengths of 10 or less are preferred, gap lengths of 5 or less are more preferred, and gap lengths of 2 or less still more preferred.

Antisense DNAs in the present invention are used to produce the corresponding antisense RNAs. An antisense RNA is an RNA which is produced with the nucleotide bases in the reverse or opposite order for expression. Such antisense RNAs are well known. *See, e.g*., U.S. Patent No. 4,801,540 to Calgene Inc. In general, the antisense RNA will be at least 15 nucleotides in length, and more typically at least 50 nucleotides in length. The antisense RNA may include an intron-exon junction (i.e., one, two, or three nucleotides on either or both sides of the intron-exon junction). Antisense RNAs which include an intron-exon junction are constructed with reference to a genomic DNA sequence.

Sense DNAs employed in carrying out the present invention are of a length sufficient to, when expressed in a plant cell, supress the native expression of a nematode-inducible transmembrane pore protein as described herein in that plant cell. Such sense DNAs may be essentially an entire genomic or complementary DNA encoding the nematode-inducible transmembrane pore protein or a fragment thereof, with such fragments typically being at least 15 nucleotides in length.

In an alternate embodiment of the present invention, the sense or antisense DNA in the construct is replaced with a DNA encoding an enzymatic RNA molecule (*i.e*., a "ribozyme"), which enzymatic RNA molecule is directed against (*i.e*., cleaves) the mRNA transcript of a DNA encoding a nematode-inducible transmembrane pore protein as described hereinabove. DNA encoding enzymatic RNA molecules may be produced in accordance with known techniques. *See, e.g*., T. Cech et al., U.S. Patent No. 4,987,071 (the disclosure of which is to be incorporated herein by reference). Production of such an enzymatic RNA molecule and disruption of pore protein production combats the infection of plants by nematodes in essentially the same manner as production of an antisense RNA molecule: that is, by disrupting translation of mRNA in the cell which produces the pore protein.

Promoters employed in carrying out the present invention may be constitutively active promoters. Numerous constitutively active promoters which are operable in plants are available. A prefered example is the cauliflower Mosaic Virus (CaMV) 35S promoter. In the alternative, the promoter may be a root-specific promoter or a nematode-responsive element, as explained in greater detail below.

Promoters which are selectively active in plant root tissue cells employed in carrying out the present invention include DNAs homologous to, and having essentially the same biological properties as, the Tobacco RB7 root-specific gene promoter disclosed herein as SEQ ID NO:4. This definition is intended to encompass natural allelic variations therein. Such elements may be of any species of origin, including tobacco, soybean, potato, peanuts, pineapple, cotton, and vegetable crops, but preferably are of dicot origin. Thus, DNA sequences which hybridize to DNA of SEQ ID NO:4 and contain a root-specific gene promoter may also be employed in carrying out the present invention. Conditions which will permit other DNA sequences which code for a such an element to hybridize to a DNA having the sequence given as SEQ ID NO:4 can be determined in a routine manner. For example, hybridization of such sequences may be carried out under conditions as given above in connection with nematode-inducible transmembrane pore proteins. Such sequences will generally be at least 75% homologous, 80% homologous, 85% homologous, 90% homologous, or even 95% homologous or more with the sequence given herein as SEQ ID NO:4. Gaps may be introduced to maximize homology when determining homology, as discussed above. In addition, homology may be determined with respect to a 10 to 15 or even 25 or 50 base segment of a DNA having the sequence of SEQ ID NO:5 and capable of directing nematode-responsive transcription of a downstream DNA sequence (i.e., a structural gene or an antisense DNA) in a plant cell. By "base segment" is meant a continuous portion thereof which is of the indicated number of nucleotides in length.

Nematode-responsive elements employed in carrying out the present invention include DNAs homologous to, and having essentially the same biological properties as, the Tobacco RB7 nematode-responsive element disclosed herein as SEQ ID NO:5. This definition is intended to encompass natural allelic variations therein. Such elements may again be of any species of origin, including tobacco, soybean, potato, peanuts, pineapple, cotton, and vegetable crops, but preferably are of dicot origin. Thus, DNA sequences which hybridize to DNA of SEQ ID NO:5 and contain a nematode-responsive element may also be employed in carrying out the present invention. Conditions which will permit other DNA sequences which code for a such an element to hybridize to a DNA having the sequence given as SEQ ID NO:5 can again be determined in a routine manner. For example, hybridization of such sequences may be carried out under conditions as given above in connection with nematode-inducible transmembrane pore proteins. Such sequences will generally be at least 75% homologous, 80% homologous, 85% homologous, 90% homologous, or even 95% homologous or more with the sequence given herein as SEQ ID NO:5. Gaps may be introduced to maximize homology when determining homology, as discussed above. In addition, homology may be determined with respect to a 10 to 15 or even 25 or 50 base segment of a DNA having the sequence of SEQ ID NO:5 and capable of directing nematode-responsive transcription of a downstream DNA sequence (i.e., a structural gene or an antisense DNA) in a plant cell.

DNA constructs, or "transcription cassettes," of the present invention include, 5' to 3' in the direction of transcription, a promoter as discussed above, a DNA operatively associated with the promoter, and, optionally, a termination sequence including stop signal for RNA polymerase and a polyadenylation signal for polyadenylase. All of these regulatory regions should be capable of operating in the cells of the tissue to be transformed. Any suitable termination signal may be employed in carrying out the present invention, examples thereof including, but not limited to, the *nos* terminator, the CaMV terminator, or native termination signals derived from the same gene as the transcriptional initiation region or derived from a different gene. The term "operatively associated," as used herein, refers to DNA sequences on a single DNA molecule which are associated so that the function of one is affected by the other. Thus, a promoter is operatively associated with a DNA when it is capable of affecting the transcription of that DNA (i.e., the DNA is under the transcriptional control of the promoter). The promoter is said to be "upstream" from the DNA, which is in turn said to be "downstream" from the promoter.

The transcription cassette may be provided in a DNA construct which also has at least one replication system. For convenience, it is common to have a replication system functional in *Escherichia coli*, such as ColE1, pSC101, pACYC184, or the like. In this manner, at each stage after each manipulation, the resulting construct may be cloned, sequenced, and the correctness of the manipulation determined. In addition, or in place of the *E. coli* replication system, a broad host range replication system may be employed, such as the replication systems of the P-1 incompatibility plasmids, e.g., pRK290. In addition to the replication system, there will frequently be at least one marker present, which may be useful in one or more hosts, or different markers for individual hosts. That is, one marker may be employed for selection in a prokaryotic host, while another marker may be employed for selection in a eukaryotic host, particularly the plant host. The markers may be protection against a biocide, such as antibiotics, toxins, heavy metals, or the like; provide complementation, by imparting prototrophy to an auxotrophic host: or provide a visible phenotype through the production of a novel compound in the plant. Exemplary genes which may be employed include neomycin phosphotransferase (NPTII), hygromycin phosphotransferase (HPT), chloramphenicol acetyltransferase (CAT), nitrilase, and the gentamicin resistance gene. For plant host selection, non-limiting examples of suitable markers are NPTII, providing kanamycin resistance or G418 resistance, HPT, providing hygromycin resistance, and the mutated aroA gene, providing glyphosate resistance.

The various fragments comprising the various constructs, transcription cassettes, markers, and the like may be introduced consecutively by restriction enzyme cleavage of an appropriate replication system, and insertion of the particular construct or fragment into the available site. After ligation and cloning the DNA construct may be isolated for further manipulation. All of these techniques are amply exemplified in the literature and find particular exemplification in J. Sambrook et al., Molecular Cloning, A Laboratory Manual (2d Ed. 1989) (Cold Spring Harbor Laboratory).

Vectors which may be used to transform plant tissue with DNA constructs of the present invention include both *Agrobacterium* vectors and ballistic vectors, as well as vectors suitable for DNA-mediated transformation.

Methods of making recombinant nematode-resistant plants of the invention, in general, involve providing a plant cell capable of regeneration (the plant cell typically residing in a tissue capable of regeneration). The plant cell is then transformed with a DNA construct comprising a transcription cassette of the present invention (as described herein) and a recombinant nematode-resistant plant regenerated from the transformed plant cell. As explained below, the transforming step is carried out by bombarding the plant cell with microparticles carrying the transcription cassette, by infecting the cell with an *Agrobacterium tumefaciens* containing a Ti plasmid carrying the transcription cassette, or any other technique suitable for the production of a transgenic plant.

Numerous Agrobacterium vector systems useful in carrying out the present invention are known. For example, U.S. Patent No. 4,459,355 discloses a method for transforming susceptible plants, including dicots, with an *Agrobacterium* strain containing the Ti plasmid. The transformation of woody plants with an *Agrobacterium* vector is disclosed in U.S. Patent No. 4,795,855. Further, U.S. Patent No. 4,940,838 to Schilperoort et al. discloses a binary *Agrobacterium* vector (i.e., one in which the *Agrobacterium* contains one plasmid having the vir region of a Ti plasmid but no T region, and a second plasmid having a T region but no vir region) useful in carrying out the present invention.

Microparticles carrying a DNA construct of the present invention, which microparticle is suitable for the ballistic transformation of a plant cell, are also useful for making transformed plants of the present invention. The microparticle is propelled into a plant cell to produce a transformed plant cell, and a plant is regenerated from the transformed plant cell. Any suitable ballistic cell transformation methodology and apparatus can be used in practicing the present invention. Exemplary apparatus and procedures are disclosed in Sanford and Wolf, U.S. Patent No. 4,945,050, and in Christou et al., U.S. Patent No. 5,015,580. When using ballistic transformation procedures, the transcription cassette may be incorporated into a plasmid capable of replicating in or integrating into the cell to be transformed. Examples of microparticles suitable for use in such systems include 1 to 5 µm gold spheres. The DNA construct may be deposited on the microparticle by any suitable technique, such as by precipitation.

Plant species may be transformed with the DNA construct of the present invention by the DNA-mediated transformation of plant cell protoplasts and subsequent regeneration of the plant from the transformed protoplasts in accordance with procedures well known in the art.

Any plant tissue capable of subsequent clonal propagation, whether by organogenesis or embryogenesis, may be transformed with a vector of the present invention. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristems, axillary buds, and root meristems), and induced meristem tissue (e.g., cotyledon meristem and hypocotyl meristem).

Plants of the present invention may take a variety of forms. The plants may be chimeras of transformed cells and non-transformed cells; the plants may be clonal transformants (e.g., all cells transformed to contain the transcription cassette); the plants may comprise grafts of transformed and untransformed tissues (e.g., a transformed root stock grafted to an untransformed scion in citrus species). The transformed plants may be propagated by a variety of means, such as by clonal propagation or classical breeding techniques. For example, first generation (or T1) transformed plants may be selfed to give homozygous second generation (or T2) transformed plants, and the T2 plants further propagated through classical breeding techniques. A dominant selectable marker (such as *npt*II) can be associated with the transcription cassette to assist in breeding.

Some plants-parasitic nematodes from which plants may be protected by the present invention, and the corresponding plants which may be employed in practicing the present invention, are as follows: Alfalfa: *Ditylenchus dipsaci, Meloidogyne hapla, Meloidogyne incognita, Meloidogyne javanica, Pratylenchus* spp., *Paratylenchus* spp., and *Xiphinema* spp.; Banana: *Radopholus similis, Helicotylenchus multicinctus, Meloidogyne incognita, M. arenaria, M. javanica, Pratylenchus coffeae,* and *Rotylenchulus reniformis*; Beans & peas: *Meloidogyne spp., Heterodera* spp., *Belonolaimus* spp., *Helicotylenchus* spp., *Rotylenchulus reniformis, Paratrichodorus anemones,* and Trichodorus spp.; cassava: *Rotylenchulus reniformis, Meloidogyne spp.* cereals: *Anguina tritici* (Emmer, rye, spelt wheat), *Bidera avenae* (oat, wheat), *Ditylenchus dipsaci* (rye, oat), *Subanguina radicicola* (oat, barley, wheat, *rye), Meloidogyne naasi* (barley, wheat, rye), *Pratylenchus* spp. (oat, wheat, barley, rye), *Paratylenchus* spp. (wheat), *Tylenchorhynchus* spp. (wheat, oat); chickpea: *Heterodera cajani, Rotylenchulus reniformis, Hoplolaimus seinhorsti, Meloidogyne* spp., *Pratylenchus* spp.; Citrus: *Tylenchulus semipenetrans, Radopholus similis, Radopholus citrophilus* (Florida only), *Hemicycliophora* arenaria, *Pratylenchus* spp., *Meloidogyne* spp., *Bolonolaimus longicaudatus* (Florida only), *Trichodorus, Paratrichodorus, Xiphinema* spp.; clover: *Meloidogyne* spp., *Heterodera trifolii*; *coconut*: *Rhadinaphelenchus cocophilus;* coffee: *Meloidogyne incognita* (Most important in Brazil), *M. exigua* (widespread), *Pratylenchus coffeae, Pratylenchus brachyurus, Radopholus similis, Rotylenchulus reniformis, Helicotylenchus* spp.; corn: *Pratylenchus* spp., *Paratrichodorus minor, Longidorus* spp., *Hoplolaimus columbus;* cotton: *Meloidogyne incognita, Belonolaimus longicaudatus, Rotylenchulus reniformis, Hoplolaimus galeatus, Pratylenchus* spp., *Tylenchorhynchus* spp., *Paratrichodorus* minor; grapes: *Xiphinema* spp., *Pratylenchus* vulnus, *Meloidogyne* spp., *Tylenchulus semipenetrans, Rotylenchulus reniformis; grasses: Pratylenchus* spp., *Longidorus* spp., *Paratrichodorus christiei, Xiphinema* spp., *Ditylenchus* spp.; peanut: *Pratylenchus* spp., *Meloidogyne hapla., Meloidogyne arenaria, Criconemella* spp., *Belonolaimus longicaudatus* (in Eastern United States); pigeonpea: *Heterodera cajani*, *Rotylenchulus reniformis, Hoplolaimus seinhorsti, Meloidogyne* spp., *Pratylenchus* spp.; pineapple: *Paratrichodorus christiei, Criconemella* spp., *Meloidogyne* spp., *Rotylenchulus reniformis, Helicotylenchus* spp., *Pratylenchus* spp., *Paratylenchus* spp.; potato: *Globodera rostochiensis, Globodera pallida, Meloidogyne* spp., *Pratylenchus* spp., *Trichodorus primitivus, Ditylenchus* spp., *Paratrichodorus* spp., *Nacoabbus aberrans*; rice: *Aphelenchiodes besseyi, Ditylenchus angustus, Hirchmanniella* spp., *Heterodera* oryzae, *Meloidogyne* spp. small fruits: *Meloidogyne* spp.; *Pratylenchus* spp., *Xiphinema* spp., *Longidorus* spp., *Paratrichodorus christiel, Aphelenchoides* spp. (strawberry); soybean: *Heterodera glycines, Meloidogyne incognita, Meloidogyne javanica,* *Belonolaimus* spp., *Hoplolaimus columbus*; sugar beet: *Heterodera schachtii, Ditylenchus dipsaci, Meloidogyne* spp., *Nacobbus aberrans, Trichodorus* spp., *Longidorus* spp., *Paratrichodorus* spp.; sugar cane: *Meloidogyne* spp., Pratylenchus spp., *Radopholus* spp., *Heterodera* spp., *Hoplolaimus* spp., *Helicotylenchus* spp., *Scutellonema* spp., *Belonolaimus* spp., *Tylenchorhynchus* spp., *Xiphinema* spp., *Longidorus* spp., Paratrichodorus spp.; tea: *Meloidogyne* spp., *Pratylenchus* spp., *Radopholus* similis, *Hemicriconemoides kanayaensis, Helicotylenchus* spp., *Paratylenchus curvitatus*; tobacco: *Meloidogyne* spp., *Pratylenchus* spp., *Tylenchorhynchus claytoni, Globodera tabacum, Trichodorus* spp., *Xiphinema americanum, Ditylenchus dipsaci* (Europe only), *Paratrichodorus spp.;* tomato: *Pratylenchus* spp., *Meloidogyne* spp.; tree fruits: *Pratylenchus* spp. (apple, pear, stone fruits), *Paratylenchus* spp. (apple, pear), *Xiphinema* spp. (pear, cherry, peach), *Cacopaurus pestis* (walnut), *Meloidogyne* spp. (stone fruits, apple, etc.), *Longidorus* spp. (cherry), *Criconemella* spp. (peach), and *Tylenchulus* spp. (olive).

In view of the foregoing, it will be apparent that plants which may be employed in practicing the present invention include (but are not limited to) tobacco (*Nicotiana tabacum*), potato (*Solanum tuberosum*), soybean (*glycine max*), peanuts (*Arachis hypogaea*), cotton (*Gossypium hirsutum*), cassava (*Manihot esculenta*), coffee (*Cofea* spp.), coconut (*Cocos nucifera*), pineapple (*Ananas comosus*), citrus trees *(Citrus* spp.), banana *(Musa* spp.), corn (*Zea mays*), wheat, oats, rye, barley, rice, and vegetables such as green beans (*Phaseolus vulgaris*), lima beans (*Phaseolus limensis*), and peas (*Lathyrus* spp.). Thus, an illustrative category of plants which may be used to practice the present invention are the dicots, and a more particular category of plants which may be used to practice the present invention are the members of the family Solanacae.

In practice, a crop comprising a plurality of plants of the invention are planted together in an agricultural field. By "agricultural field", we mean a common plot of soil or a greenhouse, with the determinative feature typically being that a common population of nematodes infect that crop of plants. Thus, the present invention provides a method of combatting plant parasitic nematodes in an agricultural field, by planting the field with a crop of plants according to the invention.

The examples which follow are set forth to illustrate the present invention, and are not to be construed as limiting thereof.

### EXAMPLE 1

### Isolation and Expression of Genomic Root-Specific Clone RB7

*Nicotiana tabacum* cv Wisconsin 38 was used as the source of material for cloning and gene characterization. Genomic DNA was partially digested with Sau3A and size-fractionated on 5 to 20% potassium acetate gradients. Size fractions of 17 to 23 kb were pooled and ligated into the λ vector, EMBL3b that had been digested with *Bam*HI and *Eco*RI. See A. Frischauf et al., J. Mol. Biol. 170, 827-842 (1983). A primary library of approximately 3.5 x 10⁶ recombinants was screened by plaque hybridization. Positive clones were plaque purified. Restriction maps of the genomic clones were constructed using the rapid mapping procedure of Rachwitz et al., Gene **30**, 195-200 (1984).

Regions encoding the root-specific clones were identified by Southern blots. To further define the transcribed regions, we took advantage of the fact that the genes are expressed at high levels. Thus, probes made of cDNA of reverse transcribed poly(A+)RNA would hybridize to Southern blots of restricted genomic clones in a manner analogous to differential screening experiments. *See* F. Kilcherr, *Nature* **321,** 493-499 (1986). The clones were digested with the appropriate restriction enzymes and the fragments separated on agarose gels. These fragments were then Southern blotted to nitrocellulose filters and probed with reverse transcribed root poly(A+)RNA. The probe was primed using random hexanucleotides (Pharmacia Biochemicals, Inc.) such that the 3' termini of the mRNA molecules would not be over represented among the probe.

Clones hybridizing to each root-specific cDNA clone were plaque purified. Comparisons of the restriction maps of the genomic clones with genomic Southern hybridization experiments (not shown) reveal a good correlation of the sequences hybridizing to the root-specific cDNA clones. Clone λ5A hybridized to the cDMA clone TobRB7. This appears to be the genomic clone corresponding to TobRB7 and accordingly was designated as TobRB7-5A (SEQ ID NO:6) and used to generate the promoter sequences employed in the experiments described below. The cell membrane channel protein is set forth as SEQ ID NO:7.

### EXAMPLE 2

### Identification of a Nematode-Responsive Element Within the TobRB7 Promoter

The ability of the TobRB7 promoter region of the λ5A genomic clone to regulate the expression of a heterologous reporter gene was tested by cloning approximately 1.4 kb of 5' flanking sequence into pBI101.2 The length of the TobRB7 flanking region employed was varied to explore how various portions of the flanking region affected expression of GUS.

In brief, a TobRB7 5' flanking region was isolated from λ5A and fused with β-glucuronidase in the *Agrobacterium* binary vector, pBI 101.2. This vector contains a β-glucuronidase (GUS) reporter gene and an *npt*II selectable marker flanked by the T-DNA border sequences (R. Jefferson et al., *EMBO J*. **6,** 3901-3907 (1987)). The TobRB7 structural gene was completely removed and the TobRB7 flanking regions fused to the GUS initiating methionene codon. The construction was mobilized into an *Agrobacterium* host that carries a disarmed Ti-plasmid (LBA4404) capable of providing (*in trans*) the *vir* functions required for T-DNA transfer and integration into the plant genome, essentially as described by An et al., *in* S. Belvin and R. Schilperoot, eds., Plant Molecular Biology Manual, Martinus Nijhoff, Dordrecht, The Netherlands, pp A3-1-19 (1988). *Nicotiana tabacum* SR1 leaf discs were infected and transformants selected and regenerated as described by An et al., Plant *Physiol*. **81**, 301-305 (1986).

Whole plants or excised root and leaf tissue were assayed for GUS expression according to Jefferson et al., *supra.* For histochemical staining, plants were incubated in-the 5-bromo-4-chloro-3-indolyl β-D-glucuronide (X-GLUC) at 37°C overnight. Tissues expressing GUS activity cleave this substrate and thereby stain blue. After the incubation the tissues were bleached in 70% ethanol. GUS enzyme activities were measured using the fluorogenic assay described by Jefferson et al.

The activity of the various deletion mutants was tested. The greatest root-specific gene expression was obtained with the Δ0.6 deletion mutant (SEQ ID NO:4). only the Δ0.3 deletion mutant (SEQ ID NO:5) was inactive as a promoter, indicating that the TobRB7 promoter is found in the region extending about 800 nucleotides upstream from the TobRB7 structural gene. However, the Δ0.3 deletion mutant (SEQ ID NO:5) contains the RB7 nematode-responsive element, as discussed below.

### EXAMPLE 3

### Localization of Gene Activation in Nematode Infected Plants

Transgenic tobacco plants prepared as described in Example 2 above were infected with tobacco root-knot nematodes (*Meloidogyne incognita*) in accordance with known techniques. *See,* e.g., C. Opperman et al., *Plant Disease,* 869-871 (October 1988). Roots were stained for GUS activity (blue) and nematodes were stained red at three stages: (a) 24-48 hours post infection; (b) 7-10 days post infection; and (c) 20-25 days post infection. Nematodes were stained after GUS staining by incubating roots in 95% ethanol/glacial acetic acid (1:1) plus five drops of acid fushsin (per 100 mLs) for four hours, then destained in a saturated chloral hydrate solution for twelve hours to overnight.

GUS activity was generally found in the elongation zone of the root. At 24-48 hours post infection, second stage juvenile nematodes have penetrated the tobacco roots, are in the corticle tissue and are migrating in search of an appropriate feeding site. Juveniles in the vascular tissue at this stage have already begun to establish feeding sites. At 7-10 days post infection, swollen late second stage juveniles are seen with their heads in the feeding site. At 20-25 days post infection, adult nematodes are seen protruding from galled root tissue, with their head still embedded in the vascular tissue and the posterior exposed to allow egg deposition.

GUS activity in nematode infected root tissue of plants transformed with the various deletion mutants described in Example 2 indicated that the nematode-responsive element of the TobRB7 promoter is located in the Δ0.3 (SEQ ID NO:5) deletion mutant.

Similar results are obtained with the peanut root-knot nematode (*Meloidogyne arenaria*).

During the foregoing experiments, it was observed that duration of gene expression in nematode-infected plants was much longer than in uninfected plants, and that the regions of gene activity were no longer restricted to the elongation zone of the root. For example, in each location where a nematode was able to establish a feeding site, gene expression continued at that site for as long as 25-30 days (i.e., the duration of the nematode life cycle).

### EXAMPLE 4

### Inhibition of Nematode Feeding Site Formation by Expression of Sense or Antisense TobRB7 mRNA

This example demonstrates the ability of transgenic plants expressing sense and anti-sense TobRB7 mRNA under the control of a constitutively active promoter to interfere with the establishment of root-knot nematode feeding sites. The constructions employed are described in **Figure 1**, and the plants were prepared in essentially the same manner as described in Example 2 above. The sense DNA employed had the sequence given herein as SEQ ID NO:1, andthe antisense DNA employed had the sequence given herein as SEQ ID NO:3. The promoter employed was the Cauliflower Mosaic Virus 35S promoter, and the termination signal employed was the *nos* terminator. The constructs were transferred to the Agrobacterium binary vector pBIN19 and transgenic plants were produced in essentially the same manner as described above: tobacco leaf disks were transformed and transformants selected on kanamycin; regenerants were allowed to self and set seeds; seeds (R2) were germinated on kanamycin and segregation of the Kan^{r} marker assayed; those plants exhibiting a 3:1 segregation (*i.e*., containing a single locus of integration) were allowed to self; progeny of the R2 were germinated on kanamycin to determine those R2 progeny that were homozygous for the transgene.

The phenotypes of a large number of control, sense, and antisense plants were examined. Control plants looked like normal tobacco. Sense and antisense plants exhibited similar phenotypes: 1) long internodes, (2) narrow and pointed leaves, and (3) early flowering. These phenotypes resemble "stress" phenotypes exhibited by plants grown in suboptimal conditions, such as small pots. It appears that the "stress" phenotype in sense plants results from the phenomenon of co-suppression: a phenomenon in which plants carrying transgenes in the sense orientation show reduced, rather than increased, levels of gene expression. *See, e.g*., C. Napoli et al., *The Plant Cell* **2,** 279-289 (1990).

Transgenic plants of sense transformants, anti-sense transformants, and control transformants were infected with second-stage juveniles of *M. arenaria* in essentially the same manner as described above. Approximately 100,000 nematodes suspended in sterile water were pipetted along the roots of plants growing on agar plates. Plants were maintained in a growth chamber at 25°C. At 24 hr post infection, juveniles were observed in various stages of root penetration on all plates. Galls were visible on all treatments by 3-5 days post infection.

Roots were harvested from plates 2A, 2B, and 7 (anti-sense); 13 and 37 (sense); and 22A and 22B (control) at 21 days post-infection. Initial observations revealed substantial and extensive galling of the sense and control plants. Galls often appeared in clusters along the root. It appeared that in a number of galls, adult female nematodes had begun reproduction. In contrast, few galls were present on the anti-sense plants. Those that were present occurred singly rather than in clusters and were substantially reduced in size compared to the sense and control plants (<50% the diameter). Two of the three plates yielded no plants with visible galling at 21 days post-infection.

Roots from each treatment were stained with acid fuchsin to determine stage of nematode development and the degree of root penetration. Roots of sense and control plants were infected with numerous nematodes in various stages of development. Mature females were observed in several galls and egg production appeared to have been initiated. Galls contained numerous nematodes. Other stages observed included vermiform second-stage juveniles, swollen second-stage juveniles, and third/fourth stage juveniles. No adult males were observed within roots or on plates. Far fewer nematodes were observed in anti-sense plants. Those that were present were mostly veriform or swollen second-stage juveniles. No adult female nenatodes were found. Several adult male nematodes were observed within the roots, but not on the plate surface. Galls that were present generally contained a single nematode and tended to occur at root junctions.

### EXAMPLE 5

### Effect on Nematode Nematode Egg Mass Rating of Expression of Sense or Antisense TobRB7 mRNA under The Control of a constitutive Promoter

Transgenic tobacco plants expressing sense or antisense TobRB7 mRNA prepared as described above were infected with tobacco root-knot nematodes (*Meloidogyne incognita*) in accordance with known techniques. See, e.g., C. Opperman et al., *Plant Disease,* 869-871 (October 1988). 63 days after infection, roots were harvested, egg masses were stained with Phloxine B to facilitate counting in accordance with known techniques and egg masses counted. Both sense and antisense plants were found resistant to nematodes. These data are given in **Table 1** below.

**TABLE 1:**

| **Egg Mass Ratings at 63 Days After Infection** | | | |
|---|---|---|---|
| Transformant Line | Egg Mass Rating | Number of Eggs | Plant Type |
| 37 | 2.6±0.5 | 1120 | sense |
| 6 | 3.6±1.0 | 3516 | antisense |
| 20 | 3.8±1.3 | 3270 | antisense |
| 2 | 4.0±1.0 | NA | antisense |
| 13 | 4.3±0.5 | 5400 | sense |
| 34 | 4.4±0.7 | 4594 | sense |
| 36 | 4.5±0.8 | 6980 | sense |
| 21 | 4.6±0.5 | 5300 | control |
| 22 | 4.7±0.5 | 6000 | control |

Egg Mass Rating: 0-no egg masses; 1=<10 egg masses; 2=10-50 egg masses; 3=50-150 egg masses; 4=150-300 egg masses; 5=>300 egg masses. NA=not available.

### EXAMPLE 6

### Inhibition of Nematode Feeding Site Formation by Expression of Sense or Antisense TobRB7 mRNA under The Control of a Nematode-Responsive Element or a Root-Specific Gene Promoter

Transgenic plants expressing sense anti-sense TobRB7 mRNA under the control of a promoter comprising a root specific gene promoter or a nematode-responsive element interfere with the establishment of root-knot nematode feeding sites. The constructions employed are described in Figure 2. Sense, antisense, and control plants were produced in essentially the same manner as described in Example 4 above, except that the root specific promoter described above and having the sequence given in SEQ ID NO:4 was employed in place of the CaMV 35S promoter. Additionally, sense, antisense, and control plants were produced in essentially the same manner as described in Example 4 above, except that the nematode-responsive element described above and having the sequence given herein as SEQ ID NO:5 was employed in place of the CaMV 35S promoter. Resistance to nematodes is shown in the same manner as described above.

The foregoing examples are illustrative of the present invention, and are not to be construed as limiting thereof. The invention is defined by the following claims, with equivalents of the claims to be included therein.

## Claims

1. A DNA construct comprising a transcription cassette, which construct comprises, in the 5' to 3' direction, a promoter operable in a plant cell, and a DNA comprising at least 15 nucleotides which are antisense to a DNA sequence encoding a nematode-inducible transmembrane pore protein which is selected from the group consisting of:
(a) isolated DNA having the sequence given herein as SEQ ID NO:1 or SEQ ID NO:6;
(b) isolated DNA which hybridizes to a complement of the isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein.

2. A DNA construct according to claim 1, which antisense DNA includes an intron-exon junction.

3. A DNA construct according to claim 1, which antisense DNA has the sequence given herein as SEQ ID NO: 3.

4. A DNA construct according to claim 1, which promoter is constitutively active in plant cells.

5. A DNA construct according to claim 1, which promoter is selectively active in plant root tissue cells.

6. A DNA construct according to claim 1, which promoter is a Cauliflower Mosaic Virus 35S promoter.

7. A DNA construct according to claim 1, which promoter is activated by a plant-parasitic nematode.

8. A DNA construct according to claim 1, which promoter is a nematode-responsive element selected from the group consisting of:
(i) isolated DNA having the sequence given herein as SEQ ID NO:5; and
(ii) isolated DNA which hybridizes to isolated DNA of (i) above and which encodes a nematode responsive element.

9. A DNA construct according to claim 1, which promoter is an RB7 nematode-responsive element.

10. A DNA construct according to claim 1 carried by a plant transformation vector.

11. A DNA construct according to claim 1 carried by a plant transformation vector, which plant transformation vector is an *Agrobacterium tumefaciens* vector.

12. A nematode-resistant transgenic plant comprising plant .cells containing a DNA construct comprising a transcription cassette, which construct comprises, in the 5' to 3' direction, a promoter operable in a plant cell, and a DNA comprising at least 15 nucleotides which are antisense to a DNA sequence encoding a nematode-inducible transmembrane pore protein which is selected from the group consisting of:
(a) isolated DNA having the sequence given herein as SEQ ID NO:1 or SEQ ID NO:6;
(b) isolated DNA which hybridizes to a complement of the isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein.

13. A plant according to claim 12, which plant is a dicot.

14. A plant according to claim 12, which plant is a dicot selected from the group consisting of tobacco, potato, soybean, peanuts, pineapple, and cotton.

15. A plant according to claim 12, which plant is a member of the family Solanacae.

16. A plant according to claim 12, which promoter is constitutively active in plant cells.

17. A plant according to claim 12, which promoter is selectively active in plant root tissue cells.

18. A plant according to claim 12, which promoter is activated by a plant-parasitic nematode.

19. A plant according to claim 12, which promoter is a nematode-responsive element selected from the group consisting of:
(i) isolated DNA having the sequence given herein as SEQ ID NO:5; and
(ii) isolated DNA which hybridizes to isolated DNA of (i) above and which encodes a nematode responsive element.

20. A crop comprising a plurality of plants according to claim 12.

21. A method of making a recombinant pathogen-resistant plant, said method comprising:
providing a plant cell capable of regeneration;
transforming said plant cell with a DNA construct comprising a transcription cassette, which construct comprises, in the 5' to 3' direction, a promoter operable in a plant cell, and a DNA comprising at least 15 nucleotides which are antisense to a DNA sequence encoding a nematode-inducible transmembrane pore protein which is selected from the group consisting of:
(a) isolated DNA having the sequence given herein as SEQ ID NO:1 or SEQ ID NO:6;
(b) isolated DNA which hybridizes to a complement of isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein; and then
regenerating a recombinant nematode-resistant plant from said transformed plant cell.

22. A method according to claim 21, wherein said plant cell resides in a plant tissue capable of regeneration.

23. A method according to claim 21, wherein said transforming step is carried out by bombarding said plant cell with microparticles carrying said transcription cassette.

24. A method according to claim 21, wherein said transforming step is carried out by infecting said cells with an *Agrobacterium tumefaciens* containing a Ti plasmid carrying said transcription cassette.

25. A DNA construct comprising a transcription cassette, which construct comprises, in the 5' to 3' direction, a promoter operable in a plant cell, and a DNA encoding an enzymatic RNA molecule directed against the mRNA transcript of a DNA sequence encoding a nematode-inducible transmembrane pore protein, said DNA sequence encoding a nematode-inducible transmembrane pore protein selected from the group consisting of:
(a) isolated DNA having the sequence given herein as SEQ ID NO:1 or SEQ ID NO:6;
(b) isolated DNA which hybridizes to a complement of the isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein.

26. A DNA construct according to claim 25, which promoter is constitutively active in plant cells.

27. A DNA construct according to claim 25, which promoter is selectively active in plant root tissue cells.

28. A DNA construct according to claim 25, which promoter is activated by a plant-parasitic nematode.

29. A DNA construct according to claim 25, which promoter is a nematode-responsive element selected from the group consisting of:
(i) isolated DNA having the sequence given herein as SEQ ID NO:5; and
(ii) isolated DNA which hybridizes to isolated DNA of (i) above and which encodes a nematode responsive element.

30. A nematode-resistant transgenic plant comprising plant cells containing a DNA construct according to claim 25.

31. A crop comprising a plurality of plants according to claim 30 .

32. A method of making a recombinant pathogen-resistant plant, said method comprising:
providing a plant cell capable of regeneration;
transforming said plant cell with a DNA construct according to claim 25; and then
regenerating a recombinant nematode-resistant plant from said transformed plant cell.

33. Use of DNA construct for conferring nematode resistance in a plant, said DNA construct comprising a transcription cassette, which construct comprises, in the 5' to 3' direction, a promoter operable in a plant cell, and a DNA comprising at least 15 nucleotides which are antisense to a DNA sequence encoding a nematode-inducible transmembrane pore protein which is selected from the group consisting of:
(a) isolated DNA having the sequence given herein as SEQ ID NO:1 or SEQ ID NO:6;
(b) isolated DNA which hybridizes to a complement of the isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein.

34. Use of a DNA construct according to claim 33, which DNA comprising at least 15 nucleotides of a DNA encoding a nematode-inducible transmembrane pore protein is an sense DNA in the proper orientation for expression.

35. Use of a DNA construct according to claim 33, which DNA comprising at least 15 nucleotides of a DNA encoding a nematode-inducible transmembrane pore protein is an antisense DNA in the opposite orientation for expression.

36. Use of a DNA construct according to claim 35, which antisense DNA includes an intron-exon junction.

37. Use of a DNA construct according to claim 35, which antisense DNA has the sequence given herein as SEQ ID NO:3.

38. Use of a DNA construct according to claim 33, which promoter is constitutively active in plant cells.

39. Use of a DNA construct according to claim 33, which promoter is selectively active in plant root tissue cells.

40. Use of a DNA construct according to claim 33, which promoter is a Cauliflower Mosaic Virus 35S promoter.

41. Use of a DNA construct according to claim 33, which promoter is activated by a plant-parasitic nematode.

42. Use of a DNA construct according to claim 33, which promoter is a nematode-responsive element selected from the group consisting of:
(i) isolated DNA having the sequence given herein as SEQ ID NO:5; and
(ii) isolated DNA which hybridizes to isolated DNA of (i) above and which encodes a nematode responsive element.

43. Use of a DNA construct according to claim 33, which promoter is an RB7 nematode-responsive element.

44. Use of a DNA construct according to claim 33 carried by a plant transformation vector.

45. Use of a DNA construct according to claim 33 carried by a plant transformation vector, which plant transformation vector is an Agrobacterium *tumefaciens* vector.

46. Use of a DNA construct for conferring nematode resistance in a plant, said DNA construct comprising a transcription cassette, which construct comprises, in the 5' to 3' direction, a promoter operable in a plant cell, and a DNA encoding an enzymatic RNA molecule directed against the mRNA transcript of a DNA sequence encoding a nematode-inducible transmembrane pore protein, said DNA sequence encoding a nematode-inducible transmembrane pore protein selected from the group consisting of:
(a) isolated DNA having the sequence given herein as SEQ ID NO:1 or SEQ ID NO:6;
(b) isolated DNA which hybridizes to a complement of the isolated DNA of (a) above and which encodes a nematode inducible transmembrane pore protein; and
(c) isolated DNA differing from the isolated DNAs of (a) and (b) above in nucleotide sequence due to the degeneracy of the genetic code, and which encode a nematode-inducible transmembrane pore protein.

47. Use of a DNA construct according to claim 46, which promoter is constitutively active in plant cells.

48. Use of a DNA construct according to claim 46, which promoter is selectively active in plant root tissue cells.

49. Use of a DNA construct according to claim 46, which promoter is activated by a plant-parasitic nematode.

50. Use of a DNA construct according to claim 46, which promoter is a nematode-responsive element selected from the group consisting of:
(i) isolated DNA having the sequence given herein as SEQ ID NO:5; and
(ii) isolated DNA which hybridizes to isolated DNA of (i) above and which encodes a nematode responsive element.

## Patentansprüche

1. DNA-Konstrukt, aufweisend eine Transkriptionskassette, welches Konstrukt aufweist: in der 5'- bis 3'-Richtung deinen in einer Pflanzenzelle operablen Promotor und eine DNA, die mindestens 15 Nucleotide aufweist, die zu einer DNA-Sequenz antisense sind, die ein nematodeninduzierbares, transmembranöses Porenprotein codiert, das ausgewählt ist aus der Gruppe, bestehend aus:
(a) isolierter DNA, die über die hierin als SEQ ID NO:1 oder SEQ ID NO:6 angegebene Sequenz verfügt;
(b) isolierter DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeninduzierbares, transmembranöses Porenprotein codiert.

2. DNA-Konstrukt nach Anspruch 1, wobei die Antisense-DNA eine Intron-Exon-Verknüpfung einschließt.

3. DNA-Konstrukt Dach Anspruch, 1, wobei die Antisense-DNA, die hierin als SEQ ID NO:3 angegebene Sequenz hat.

4. DNA-Konstrukt nach Anspruch 1, wobei der Promotor in Pflanzenzellen konstitutiv aktiv ist.

5. DNA-Konstrukt nach Anspruch 1, wobei der Promotor in Gewebezellen von Pflanzenwurzeln selektiv aktiv ist.

6. DNA-Konstrukt nach Anspruch 1, wobei der Promotor ein Blumenkohlmosaik-Virus 35S-Promotor ist.

7. DNA-Konstrukt nach Anspruch 1, wobei der Promotor durch eine pflanzenparasitäre Nematode aktiviert wird.

8. DNA-Konstrukt nach Anspruch 1, wobei der Promotor ein auf Nematoden reagierendes Element ist, ausgewählt aus der Gruppe, bestehend aus:
(i) isolierter DNA mit der hierin als SEQ ID NO:5 angegebenen Sequenz; und
(ii) isolierter DNA, die zu isolierter DNA von (i) vorstehend hybridisiert wird und die ein auf Nematoden reagierendes Element codiert.

9. DNA-Konstrukt nach Anspruch 1, wobei der Promotor ein auf Nematoden reagierendes RB7-Element ist

10. DNA-Konstrukt nach Anspruch 1, getragen von einem Pflanzen-Transformationsvektor.

11. DNA-Konsfrukt nach Anspruch 1, getragen von einem Pflanzen-Transformationsvektor, wobei der Pflanzen-Transformationsvektor ein *Agrobacterium tumefaciens*-Vektor ist.

12. Nematodenresistente, transgene Pflanze, aufweisend Pflanzenzellen, die ein DNA-Konstrukt enthalten, das eine Transkriptionskassette aufweist, wobei das Konstrukt in der 5'- bis 3'-Richtung einen in einer Pflanzenzelle operablen Promotor aufweist und eine DNA, die mindestens 15 Nucleotide aufweist, die in antisense zu einer DNA-Sequenz sind, die ein namatodeninduzierbares, transmembranöses Porenprotein codiert, ausgewählt aus der Gruppe, bestehend aus:
(a) isolierter DNA, die über die hierin als SEQ ID NO:1 oder SEQ ID NO:6 gegebene Sequenz verfügt;
(b) isolierter DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeniduzierbares, transmembranöses Porenprotein codiert.

13. Pflanze nach Anspruch 12, wobei die Pflanze eine zweikeimblättrige Pflanze ist.

14. Pflanze nach Anspruch 12, wobei die Pflanze eine zweikeimblättrige Pflanze ist, ausgewählt aus der Gruppe, bestehend aus Tabak, Kartoffel, Sojabohne, Erdnuss, Ananas und Baumwolle.

15. Pflanze nach Anspruch 12, wobei die Pflanze ein Vertreter der Familie der Solanaceae ist.

16. Pflanze nach Anspruch 12, wobei der Promotor in Pflanzenzellen konstitutiv aktiv ist.

17. Pflanze nach Anspruch 12, wobei der Promotor in Gewebezellen von Pflanzenwurzeln selektiv aktiv ist.

18. Pflanze nach Anspruch 12, wobei der Promotor durch eine pflanzenparasitäre Nematode aktiviert wird.

19. Pflanze nach Anspruch 12, wobei der Promotor ein auf Nematoden reagierendes Element ist, ausgewählt aus der Gruppe, bestehend aus:
(i) isolierter DNA mit der hierin als SEQ ID NO:5 angegebenen Sequenz; und
(ii) isolierter DNA, die zu isolierter DNA von (i) vorstehend hybridisiert wird und die ein auf Nematoden reagierendes Element codiert.

20. Feldfrucht, umfassend eine Vielzahl von Pflanzen nach Anspruch 12.

21. Verfahren zum Herstellen einer rekombinanten pathogenresistenten Pflanze, welches Verfahren umfasst:
Bereitstellen einer Pflanzenzelle, die zur Regeneration in der Lage ist;
Transformieren dieser Pflanzenzelle mit einem DNA-Konstrukt, aufweisend eine Transkriptionskassette, wobei das Konstrukt in 5'- bis 3'-Richtung einen in einer Pflanzenzelle operablen Promotor aufweist und eine DNA, aufweisend mindestens 15 Nucleotide, die zu einer DNA-Sequenz antisense sind, die ein nematodeninduzierbares transmembranöses Porenprotein codiert, ausgewählt aus der Gruppe, bestehend aus:
(a) isolierter DNA, die über die hierin als SEQ ID NO:1 oder SEQ ID NO:6 angegebene Sequenz verfügt;
(b) isolierter DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeninduzierbares, transmembranöses Porenprotein codiert; und anschließend
Regenerieren einer rekombinanten, nematodenresistenten Pflanze aus der transformierten Pflanzenzelle.

22. Verfahren nach Anspruch 21, bei welchem die Pflanzenzelle sich in einem Pflanzengewebe befindet, das zur Regeneration in der Lage ist.

23. Verfahren nach Anspruch 21, bei welchem der Schritt des Transformierens ausgeführt wird durch Beschuss der Pflanzenzelle mit Mikropartikeln, die die Transkriptionskassette tragen.

24. Verfahren nach Anspruch 21, bei welchem der Schritt des Transformierens ausgeführt wird durch Infizieren der Zellen mit einem *Agrobacterium tumefaciens*, das ein Ti-Plasmid enthält, welches die Transkriptionskassette trägt.

25. DNA-Konstrukt, auf weisend eine Transkriptionskassette, wobei das Konstrukt in 5'- bis 3'-Richtung einen in einer Pflanzenzelle operablen Promotor aufweist und eine DNA, die ein enzymatisches RNA-Molekül codiert, das gegen das mRNA-Transkript einer DNA-Sequenz gerichtet ist, die ein nematodeninduzierbares, transmembranöses Porenprotein codiert, wobei die das nematodeninduzierbare, transmembranöse Porenprotein-codierende DNA-Sequenz ausgewählt wird aus der Gruppe, bestehend aus:
(a) isolierter DNA, die über die hierin als SEQ ID NO:1 oder SEQ ID NO:6 angegebene Sequenz verfügt;
(b) isolierte DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeninduzierbares, transmembranöses Porenprotein codiert.

26. DNA-Konstrukt nach Anspruch 25, wobei der Promotor in Pflanzenzellen konstitutiv aktiv ist.

27. DNA-Konstrukt nach Anspruch 25, wobei der Promotor in Gewebezellen von Pflanzenwurzeln selektiv aktiv ist.

28. DNA-Konstrukt nach Anspruch 25, wobei der Promotor durch eine pflanzenparasitäre Nematode aktiviert wird.

29. DNA-Konstrukt nach Anspruch 25, wobei der Promotor ein auf Nematoden reagierendes Element ist, ausgewählt aus der Gruppe, bestehend aus:
(i) isolierter DNA mit der hierin als SEQ ID NO:5 angegebenen Sequenz; und
(ii) isolierter DNA, die zu isolierter DNA von (i) vorstehend hybridisiert wird und die ein auf Nematoden reagierendes Element codiert.

30. Nematodenresistente, transgene Pflanze, aufweisend Pflanzenzellen, die ein DNA-Konstrukt nach Anspruch 25 enthalten.

31. Feldfrucht, umfassend eine Vielzahl von Pflanzen nach Anspruch 30.

32. Verfahren zum Herstellen einer rekombinanten, pathogenresistenten Pflanze, welches Verfahren umfasst:
Bereitstellen einer Pflanzenzelle, die zur Regeneration in der Lage ist;
Transformieren dieser Pflanzenzelle mit einem DNA-Konstrukt nach Anspruch 25 und anschließend
Regenerieren einer rekombinanten, nematodenresistenten Pflanze aus der transformierten Pflanzenzelle.

33. Verwendung eines DNA-Konstruktes, um einer Pflanze Nematodenresistenz zu vermitteln, wobei das DNA-Konstrukt eine Transkriptionskassette aufweist und das Konstrukt in 5'- bis 3'-Richtung einen in einer Pflanzenzelle operablen Promotor aufweist und eine DNA, die mindestens 15 Nucleotide aufweist, die in antisense zu einer DNA-Sequenz sind, die ein nematodeninduzierbares, transmembranöses Porenprotein codiert, die ausgewählt ist aus der Gruppe, bestehend aus:
(a) isolierter DNA, die über die hierin als SEQ ID NO:1 oder SEQ ID NO:6 angegebene Sequenz verfügt;
(b) isolierter DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeninduzierbares, transmembranöses Porenprotein codiert.

34. Verwendung eines DNA-Konstrukts nach Anspruch 33, wobei die DNA, die mindestens 15 Nucleotide einer DNA aufweist, die ein nematodeninduzierbares, transmembranöses Porenprotein codiert, eine Sense-DNA in geeigneter Orientierung zur Expression ist.

35. Verwendung eines DNA-Konstrukts nach Anspruch 33, wobei die DNA, die mindestens 15 Nucleotide einer DNA aufweist, die ein nematodeninduzierbares, transmembranöses Porenprotein codiert, eine Antisense-DNA in der entgegengesetzten Orientierung zur Expression ist.

36. Verwendung eines DNA-Konstrukts nach Anspruch 35, wobei die Antisense-DNA eine Intron-Extron-Verknüpfung einschließt.

37. Verwendung eines DNA-Konstrukts nach Anspruch 35, wobei die Antisense-DNA, die hierin als SEQ ID NO:3 angegebene Sequenz hat.

38. Verwendung eines DNA-Konstrukts nach Anspruch 33, wobei der Promotor in Pflanzenzellen konstitutiv aktiv ist.

39. Verwendung eines DNA-Konstrukts nach Anspruch 33, wobei der Promotor in Gewebezellen von Pflanzenwurzeln selektiv aktiv ist.

40. Verwendung eines DNA-Konstrukts nach Anspruch 33, wobei der Promotor ein Blumenkohlmosaik-Virus 35S-Promotor ist.

41. Verwendung eines DNA-Konstrukts nach Anspruch 33, wobei der Promotor durch eine pflanzenparasitäre Nematode aktiviert wird.

42. Verwendung eines DNA-Konstrukts nach Anspruch 33, wobei der Promotor ein auf Nematoden reagierendes Element ist, ausgewählt aus der Gruppe, bestehend aus:
(i) isolierter DNA mit der hierin als SEQ ID NO:5 angegebenen Sequenz; und
(ii) isolierter DNA, die zu isolierter DNA von (i) vorstehend hybridisiert und die ein auf Nematoden reagierendes Element codiert.

43. Verwendung eines DNA-Konstrukts nach Anspruch 33, wobei der Promotor ein auf Nematoden reagierendes RB7-Element ist

44. Verwendung eines DNA-Konstrukts nach Anspruch 33, getragen von einem Pflanzen-Transformationsvektor.

45. Verwendung eines DNA-Konstrukts nach Anspruch 33, getragen von einem Pflanzen-Transformationsvektor, wobei der Pflanzen-Transformationsvektor ein *Agrobacterium tumefaciens*-Vektor ist.

46. Verwendung eines DNA-Konstrukts, um einer Pflanze Nematodenresistenz zu vermitteln, wobei das DNA-Konstrukt eine Transkriptionskassette aufweist und das Konstrukt in 5'- bis 3'-Richtung einen in einer Pflanzenzelle operablen Promotor aufweist und eine DNA, die ein enzymatisches RNA-Molekül codiert, das gegen das mRNA-Transkript einer DNA-Sequenz gerichtet ist, die ein nematodeninduzierbares, transmembranöses Porenprotein codiert, wobei die das nematodeninduzierbare, transmembranöse Porenprotein codierende DNA-Sequenz ausgewählt ist aus der Gruppe, bestehend aus:
(a) isolierter DNA, die über die hierin als SEQ ID NO:1 oder SEQ ID NO:6 angegebene Sequenz verfügt;
(b) isolierter DNA, die zu einem Komplement der isolierten DNA von (a) vorstehend hybridisiert und die ein nematodeninduzierbares, transmembranöses Porenprotein codiert; und
(c) isolierter DNA, die sich von den isolierten DNA's von (a) und (b) vorstehend in der Nucleotid-Sequenz auf Grund der Degeneration des genetischen Codes unterscheidet und die ein nematodeninduzierbares, transmembranöses Porenprotein codiert.

47. Verwendung eines DNA-Konstrukts nach Anspruch 46, wobei der Promotor in Pflanzenzellen konstitutiv aktiv ist.

48. Verwendung eines DNA-Konstrukts nach Anspruch 46, wobei der Promotor in Gewebezellen von Pflanzenwurzeln selektiv aktiv ist.

49. Verwendung eines DNA-Konstrukts nach Anspruch 46, wobei der Promotor durch eine pflanzenparasitäre Nematode aktiviert wird.

50. Verwendung eines DNA-Konstrukts nach Anspruch 46, wobei der Promotor ein auf Nematoden reagierendes Element ist, ausgewählt aus der Gruppe, bestehend aus:
(i) isolierter DNA mit der hierin als SEQ ID NO:5 angegebenen Sequenz; und
(ii) isolierter DNA, die zu isolierter DNA von (i) vorstehend hybridisiert und die ein auf Nematoden reagierendes Element codiert.

## Revendications

1. Construction d'ADN comprenant une cassette de transcription, laquelle construction comprend, dans la direction 5' à 3', un promoteur pouvant agir dans une cellule de plante, et un ADN comprenant au moins 15 nucléotides qui sont anti-sens par rapport à une séquence d'ADN codant pour une protéine de pore transmembranaire inductible par un nématode qui est choisie dans le groupe constitué:
(a) d'un ADN isolé possédant la séquence donnée dans le présent document comme SEQ ID NO:1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence du code génétique et qui code pour une protéine de pore transmembranaire inductible par un nématode.

2. Construction d'ADN suivant la revendication 1, dont l'ADN anti-sens inclut une jonction intron-exon.

3. Construction d'ADN suivant la revendication 1, dont l'ADN anti-sens possède la séquence donnée dans le présent document comme SEQ ID NO:3.

4. Construction d'ADN suivant la revendication 1, dont le promoteur est actif constitutivement dans des cellules de plante.

5. Construction d'ADN suivant la revendication 1, dont le promoteur est actif sélectivement dans des cellules de tissu de racine de plante.

6. Construction d'ADN suivant la revendication 1, dont le promoteur est un promoteur du virus de la mosaïque du chou-fleur 35S.

7. Construction d'ADN suivant la revendication 1, dont le promoteur est activé par un nématode parasitique de plante.

8. Construction d'ADN suivant la revendication 1, dont le promoteur est un élément sensible à un nématode choisi dans le groupe constitué:
(i) d'un ADN isolé possédant la séquence donnée dans le présent document comme SEQ ID NO:5; et
(ii) d'un ADN isolé qui s'hybride à l'ADN isolé de (i) ci-dessus et qui code pour un élément sensible à un nématode.

9. Construction d'ADN suivant la revendication 1, dont le promoteur est un élément sensible à un nématode RB7.

10. Construction d'ADN suivant la revendication 1, portée par un vecteur de transformation de plante.

11. Construction d'ADN suivant la revendication 1, portée par un vecteur de transformation de plante, lequel vecteur de transformation de plante est un vecteur de *Agrobacterium tumefaciens*.

12. Plante transgénique résistant à un nématode comprenant des cellules de plante contenant une construction d'ADN comprenant une cassette de transcription, laquelle construction comprend, dans la direction 5' à 3', un promoteur pouvant agir dans une cellule de plante, et un ADN comprenant au moins 15 nucléotides qui sont anti-sens par rapport à une séquence d'ADN codant pour une protéine de pore transmembranaire inductible par un nématode qui est choisie dans le groupe constitué:
(a) d'un ADN isolé possédant la séquence donnée dans le présent document comme SEQ ID NO:1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence du code génétique et qui code pour une protéine de pore transmembranaire inductible par un nématode.

13. Plante suivant la revendication 12, laquelle plante est un dicotylédone.

14. Plante suivant la revendication 12, laquelle plante est un dicotylédone choisi dans le groupe constitué de tabac, de pomme de terre, de graines de soja, de cacahouètes, d'ananas et de coton.

15. Plante suivant la revendication 12, laquelle plante est un membre de la famille des Solanacées.

16. Plante suivant la revendication 12, dont le promoteur est actif constitutivement dans des cellules de plante.

17. Plante suivant la revendication 12, dont le promoteur est actif sélectivement dans des cellules de tissu de racine de plante.

18. Plante suivant la revendication 12, dont le promoteur est activé par un nématode parasitique de plante.

19. Plante suivant la revendication 12, dont le promoteur est un élément sensible à un nématode choisi dans le groupe constitué:
(i) d'un ADN isolé possédant la séquence donnée dans le présent document comme SEQ ID NO:5; et
(ii) d'un ADN isolé qui s'hybride à l'ADN isolé de (i) ci-dessus et qui code pour un élément sensible à un nématode.

20. Récolte comprenant une pluralité de plantes suivant la revendication 12.

21. Procédé pour la fabrication d'une plante recombinante résistant à un pathogène, ledit procédé comprenant:
la fourniture d'une cellule de plante apte à une régénération;
la transformation de ladite cellule de plante avec une construction d'ADN comprenant une cassette de transcription, laquelle construction comprend, dans la direction 5' à 3', un promoteur pouvant agir dans une cellule de plante, et un ADN comprenant au moins 15 nucléotides qui sont anti-sens par rapport à une séquence d'ADN codant pour une protéine de pore transmembranaire inductible par un nématode qui est choisie dans le groupe constitué:
(a) d'un ADN isolé possédant la séquence donnée dans le présent document comme SEQ ID NO:1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence .du code génétique et qui code pour une protéine de pore transmembranaire inductible par un nématode; et ensuite
la régénération d'une plante recombinante résistant à un nématode à partir de ladite cellule de plante transformée.

22. Procédé suivant la revendication 21, dans lequel ladite cellule de plante réside dans un tissu de plante apte à une régénération.

23. Procédé suivant la revendication 21, dans lequel ladite étape de transformation est réalisée en bombardant ladite cellule de plante avec des microparticules portant ladite cassette de transcription.

24. Procédé suivant la revendication 21, dans lequel ladite étape de transformation est réalisée en infectant lesdites cellules avec un *Agrobacterium tumefaciens* contenant un plasmide Ti portant ladite cassette de transcription.

25. Construction d'ADN comprenant une cassette de transcription, laquelle construction comprend, dans la direction 5' à 3', un promoteur pouvant agir dans une cellule de plante, et un ADN codant pour une molécule d'ARN enzymatique dirigée contre le transcrit d'ARNm d'une séquence d'ADN codant pour une protéine de pore transmembranaire inductible par un nématode, ladite séquence d'ADN codant pour une protéine de pore transmembranaire inductible par un nématode est choisie dans le groupe constitué:
(a) d'un ADN isolé possédant la séquence donnée dans le présent document comme SEQ ID NO.1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence du code génétique et qui code pour une protéine de pore transmembranaire inductible par un nématode.

26. Construction d'ADN suivant la revendication 25, dont le promoteur est actif constitutivement dans des cellules de plante.

27. Construction d'ADN suivant la revendication 25, dont le promoteur est actif sélectivement dans des cellules de tissu de racine de plante.

28. Construction d'ADN suivant la revendication 25, dont le promoteur est activé par un nématode parasitique de plante.

29. Construction d'ADN suivant la revendication 25, dont le promoteur est un élément sensible à un nématode choisi dans le groupe constitué:
(i) d'un ADN isolé possédant la séquence donnée dans le présent document comme SEQ ID NO:5; et
(ii) d'un ADN isolé qui s'hybride à l'ADN isolé de (i) ci-dessus et qui code pour un élément sensible à un nématode.

30. Plante transgénique résistant à un nématode comprenant des cellules de plantes contenant une construction d'ADN suivant la revendication 25.

31. Récolte comprenant une pluralité de plantes suivant la revendication 30.

32. Procédé pour la fabrication d'une plante recombinante résistant à un pathogène, ledit procédé comprenant:
la fourniture d'une cellule de plante apte à une régénération;
la transformation de ladite cellule de plante avec une construction d'ADN suivant la revendication 25; et ensuite
la régénération d'une plante recombinante résistant à un nématode à partir de ladite cellule de plante transformée.

33. Utilisation d'une construction d'ADN pour conférer une résistance à un nématode dans une plante, ladite construction d'ADN comprenant une cassette de transcription, laquelle construction comprend, dans la direction 5' à 3', un promoteur pouvant agir dans une cellule de plante, et un ADN comprenant au moins 15 nucléotides qui sont anti-sens par rapport à une séquence d'ADN codant pour une protéine de pore transmembranaire inductible par un nématode qui est choisie dans le groupe constitué:
(a) d'un ADN isolé possédant la séquence donnée dans le présent document comme SEQ ID NO:1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence du code génétique et qui code pour une protéine de pore transmembranaire inductible par un nématode.

34. Utilisation d'une construction d'ADN suivant la revendication 33, lequel ADN comprenant au moins 15 nucléotides d'un ADN codant pour une protéine de pore transmembranaire inductible par un nématode est un ADN sens dans l'orientation correcte pour une expression.

35. Utilisation d'une construction d'ADN suivant la revendication 33, lequel ADN comprenant au moins 15 nucléotides d'un ADN codant pour une protéine de pore transmembranaire inductible par un nématode est un ADN anti-sens dans l'orientation opposée pour une expression.

36. Utilisation d'une construction d'ADN suivant la revendication 35, lequel ADN anti-sens inclut une jonction intron-exon.

37. Utilisation d'une construction' d'ADN suivant la revendication 35, lequel ADN anti-sens possède la séquence donnée dans le présent document comme SEQ ID NO:3.

38. Utilisation d'une construction d'ADN suivant la revendication 33, dont le promoteur est actif constitutivement dans des cellules de plante.

39. Utilisation d'une construction d'ADN suivant la revendication 33, dont le promoteur est actif sélectivement dans des cellules de tissu de racine de plante.

40. Utilisation d'une construction d'ADN suivant la revendication 33, dont le promoteur est un promoteur du virus de la mosaïque du chou-fleur 35S.

41. Utilisation d'une construction d'ADN suivant la revendication 33, dont le promoteur est activé par un nématode parasitique de plante.

42. Utilisation d'une construction d'ADN suivant la revendication 33, dont le promoteur est un élément sensible à un nématode choisi dans le groupe constitué:
(i) d'un ADN isolé possédant la séquence donnée dans le présent document comme SEQ ID NO:5; et
(ii) d'un ADN isolé qui s'hybride à l'ADN isolé de (i) ci-dessus et qui code pour un élément sensible à un nématode.

43. Utilisation d'une construction d'ADN suivant la revendication 33, dont le promoteur est un élément sensible à un nématode RB7.

44. Utilisation d'une construction d'ADN suivant la revendication 33, portée par un vecteur de transformation de plante.

45. Utilisation d'une construction d'ADN suivant la revendication 33, portée par un vecteur de transformation de plante, lequel vecteur de transformation de plante est un vecteur de *Agrobacterium tumefaciens*.

46. Utilisation d'une construction d'ADN pour conférer une résistance à un nématode dans une plante, ladite construction d'ADN comprenant une cassette de transcription, laquelle construction comprend, dans la direction 5' à 3', un promoteur pouvant agir dans une cellule de plante, et un ADN codant pour une molécule d'ARN enzymatique dirigée contre le transcrit d'ARNm d'une séquence d'ADN codant pour une protéine de pore transmembranaire inductible par un nématode, laquelle séquence d'ADN codant pour une protéine de pore transmembranaire inductible par un nématode est choisie dans le groupe constitué:
(a) d'un ADN isolé possédant la séquence donnée dans le présent document comme SEQ ID NO:1 ou SEQ ID NO:6;
(b) d'un ADN isolé qui s'hybride à un complément de l'ADN isolé de (a) ci-dessus et qui code pour une protéine de pore transmembranaire inductible par un nématode; et
(c) d'un ADN isolé qui diffère des ADN isolés de (a) et de (b) ci-dessus dans la séquence de nucléotides à cause de la dégénérescence du code génétique et qui code pour une protéine de pore transmembranaire inductible par un nématode,

47. Utilisation d'une construction d'ADN suivant la revendication 46, dont le promoteur est actif constitutivement dans des cellules de plante.

48. Utilisation d'une construction d'ADN suivant la revendication 46, dont le promoteur est actif sélectivement dans des cellules de tissu de racine de plante.

49. Utilisation d'une construction d'ADN suivant la revendication 46. dont le promoteur est activé par un nématode parasitique de plante.

50. Utilisation d'une construction d'ADN suivant la revendication 46, dont le promoteur est un élément sensible à un nématode choisi dans le groupe constitué:
(i) d'un ADN isolé possédant la séquence donnée dans le présent document comme SEQ ID NO:5; et
(ii) d'un ADN isolé qui s'hybride à l'ADN isolé de (i) ci-dessus et qui code pour un élément sensible à un nématode.
